(19) Europäisches Patentamt
European Patent Office
Office européen des brevets

(11) Publication number : 0 400 546 B1

(12) **EUROPEAN PATENT SPECIFICATION**

(45) Date of publication of patent specification : **08.12.93 Bulletin 93/49**

(51) Int. Cl.$^5$ : **A61K 7/32**, A61K 7/40, A61K 7/06

(21) Application number : **90110100.6**

(22) Date of filing : **28.05.90**

# (54) Stable anhydrous compositions for topical delivery of active materials.

The file contains technical information submitted after the application was filed and not included in this specification

(30) Priority : **02.06.89 US 360418**

(43) Date of publication of application : **05.12.90 Bulletin 90/49**

(45) Publication of the grant of the patent : **08.12.93 Bulletin 93/49**

(84) Designated Contracting States : **AT BE CH DE DK ES FR GB GR IT LI LU NL SE**

(56) References cited :
**EP-A- 0 313 304**
**EP-A- 0 388 111**
**US-A- 3 818 105**
**US-A- 4 724 139**

(73) Proprietor : **HELENE CURTIS, INC.**
**325 North Wells Street**
**Chicago Illinois 60610 (US)**

(72) Inventor : **McCrea, Andrew D.**
**345 North Canal, Apt. 1406**
**Chicago, Illinois 60606 (US)**
Inventor : **Diulus, Michael P.**
**530 South Scoville, Apt. 2N**
**Oak Park, Illinois 60304 (US)**

(74) Representative : **Reitzner, Bruno, Dr. et al**
**Patentanwälte Dipl.-Ing. R. Splanemann Dr. B. Reitzner, Dipl.-Ing. K. Baronetzky Tal 13**
**D-80331 München (DE)**

EP 0 400 546 B1

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Jouve, 18, rue Saint-Denis, 75001 PARIS

## Description

### FIELD OF THE INVENTION

The present invention is directed to a topically-effective composition in liquid, cream or paste form. More particularly, the composition of the present invention is an unexpectedly stable suspension useful for the improved topical delivery of a topically-active compound, either cosmetic or medical, to the skin. The topically-effective composition resists phase separation; is essentially nonwhitening and nonstaining to skin and clothing after topical application; effectively delivers the topically-active compound; and exhibits superior sensory properties.

### BACKGROUND OF THE INVENTION

As set forth in the Deckner U.S. -A-4,563,346, an ideal composition for delivering a topically-active compound to skin or hair should be as stable as possible and should deliver the topically-active compound such that it adheres to the skin or hair while other non-active ingredients evaporate or are otherwise removed from the area of application. Topically-delivered active compounds, such as cosmetics, like an antiperspirant compound, and topical medications, like an antibacterial or an anti-inflammatory, traditionally have been prepared as either oil-in-water emulsions or water-in-oil emulsions. However, topically-effective compositions prepared as emulsions feel wet or oily when applied to the skin, and often remain sticky after the composition carrier evaporates. Furthermore, emulsion-type compositions require a relatively long time to dry after topical application. In addition, many emulsion-type compositions leave a white residue on contacted skin or clothing and actually stain clothing.

However, non-emulsified anhydrous compositions, like antiperspirants, are known in the art. Non-emulsified, oil-based topically-effective compositions are available, however these products often require shaking prior to each use in order to redisperse the insoluble topically-active compound that has separated from the composition. For example, U.S.-A-3,873,686 discloses an anhydrous, liquid or creme antiperspirant composition comprising an alcohol-soluble aluminum chlorhydroxide-polyol complex in an anhydrous ethanol vehicle. Similarly, U.S.-A-4,137,306 discloses the above-described anhydrous antiperspirant composition in solid stick form. U.S.-A-4,053,581; 4,065,564 and 4,073,880 disclose liquid anhydrous antiperspirant compositions useful as pump-spray or roll-on products, wherein the antiperspirant compounds are solubilized in a vehicle including ethanol and a sufficient amount of volatile and/or non-volatile silicone liquid to reduce tackiness of the antiperspirant. GB-A-2018590 describes an anhydrous antiperspirant spray composition including from 60% to 90% of a volatile cyclic silicone in order to improve composition efficacy by increasing adherence of the antiperspirant composition to the skin and hair. Nevertheless, although suspending agents are included in each of the above-cited references, shaking of the antiperspirant compositions before use is necessary in order to redisperse the separated antiperspirant compound.

Stable, non-separating antiperspirant compositions also are known in the art. U.S.-A-4,749,569 discloses an extrudable antiperspirant paste; or creme, composition stabilized against phase separation by thickening the antiperspirant composition with from 4.6% to 9.5% of a finely-divided silica and from 2% to 25% of a quaternized three-layer clay exfoliated with a polar solvent. If a finely-divided silica is used as the sole thickening, or suspending, agent an unstable product results, thereby requiring the use of an additional suspending agent, like an organoclay. However, the presence of an organoclay in an antiperspirant composition is a principal source of the whitening and staining of the skin and clothing.

U.S.-A-3,818,105 discloses that $C_{12}$ to $C_{14}$ isoparaffinic hydrocarbons, when combined with naphthenic materials, are useful in a wide range of cosmetic formulations for lubrication of the skin to achieve a quick spreading, non greasy application with evaporation of the hydrocarbon after use without a greasy buildup. While the hydrocarbons disclosed in the US-A-3,818,105 lubricate the skin for better application of the cosmetic formula, these compositions do not achieve the unexpected stability achieved in accordance with the present invention. Other compositions containing volatile hydrocarbons, such as those disclosed in U.S.-A-4,472,375, require polymeric water-soluble thickening agents, such as guar gum, to effectively stabilize the compositions.

US-A-724 139 discloses an antiperspirant stick composition comprising from 5 to 80 % of a volatile isoparaffin liquid carrier, from 5 to 60 % of one or more water-insoluble waxes and from 8 to 60 % of an active aluminum or zirconium adstringent antiperspirant salt in form of finely divided particles. The high percentage of wax makes the composition solid, and there is no problem of phase separation as it is with liquid or cream compositions.

EP-A-0 388 111 discloses low residue antiperspirant sticks which comprise a volatile silicone material, a

particular antiperspirant agent, a low melting point wax and a non-volatile paraffinic hydrocarbon fluid. Again, since there are no liquid or cream compositions, the problem of phase separation does not arise.

## SUMMARY OF THE INVENTION

The present invention is directed to a topically-effective composition in liquid, cream or paste form for application to the skin or hair comprising:

(a) from 0.01 % to 30 % by weight of a topically-active compound.

(b) from 20 % to 99 % by weight of a volatile liquid carrier which has a boiling point from 150 to 250°C if it is a polydimethyl siloxane, and from 100 to 300°C if it is a hydrocarbon;

(c) from 0.1 to 15 % by weight of a finely-divided silica;

(d) from 0.25 to 3.5 % by weight of a wax; and

(e) from 0.25 % to 4.0 % by weight of an ester including at least ten carbon atoms;

In particular, the wax (component d) and the ester (component e) may be present as a suspending wax composition in an amount of from 1 to 10 % by weight, the suspending wax composition comprising from 25 to 35 % by weight of a wax; from 30 % to 45 % by weight of a volatile silicone or a volatile hydrocarbon; and from 25 40 % by weight of an ester including at least ten carbon atoms.

Furthermore, the invention relates to a method of manufacturing a stable, topically-effective composition in liquid, cream or paste form for the topical delivery of a topically-active compound comprising:

preparing a suspending wax composition comprising from 25 % to 35 % by weight of a wax; from 30 % to 45 % by weight of a volatile solvent selected from a volatile silicone, a volatile hydrocarbon, and combinations thereof, which solvent has a boiling point from 150 to 250 °C if it is polydimethyl siloxane, and from 100 to 300°C if it is a hydrocarbon; and from 25 to 40 % by weight of an ester including at least ten carbon atoms; preparing a silicone dispersion comprising from 0.1 parts to 15 parts by weight of a finely-divided silica and from 20 parts to 95 parts by weight of a volatile liquid carrier;

combining from 1 part to 10 parts by weight of the suspending wax composition with from 0.01 parts to 30 parts by weight of a topically-active compound with a silica dispersion to or a topically-active mixture; and thoroughly admixing the topically-active mixture to form a homogeneous topically-effective composition.

Preferred embodiments of the present invention are recited in the dependent claims.

Suspending wax compositions have been used in the paint industry as a suspending agent for pigments. Unexpectedly, it has been found that including a suspending wax composition, comprising a natural or synthetic wax, like castor wax; a volatile silicone or a volatile hydrocarbon; and an ester, in an anhydrous, topically-effective composition of the present invention eliminates the need for including the traditional organoclay suspending agents. Surprisingly, it was further found that including the suspending wax composition in a topically-effective composition, such as an antiperspirant, allows lesser amounts of the traditional active antiperspirant ingredients to be included in the composition. Consequently, the texture and consistency, or feel, of the antiperspirant composition is dramatically improved by masking, reducing, or eliminating the undesirable sensory properties attributed to the traditional antiperspirant compounds, such as stickiness. Other undesirable sensory properties reduced or eliminated by the compositions of the present invention include grittiness caused by the finely-divided silica; oiliness and long-drying time caused by the use of traditional emollients and polyols; and whitening and staining of skin and clothing caused by the organoclay suspending agents.

In addition, it has been found that the addition of a cosolvent, such as a non-volatile silicone, a high molecular weight ester, a high molecular weight polyol or an oil-soluble surfactant, to an anhydrous topically-effective composition of the present invention, further reduces or eliminates phase separation by enhancing the intermolecular bond formation, and therefore the suspending ability, of the finely-divided silica. It also was found that shearing the finely-divided silica prior to adding the silica to the volatile liquid carrier vehicle of the present invention further enhanced intermolecular silica bond formation, thereby further contributing to reduced phase separation.

In one important embodiment of the present invention, the anhydrous, topically-effective composition of the present invention incorporates an astringent salt as the topically-active compound to form a stable and efficacious antiperspirant composition. In other embodiments of the present invention, the anhydrous, topically-effective composition incorporates topically effective drugs and medicaments; topical anesthetics; sunscreen agents; skin-soothing emollients and other topical cosmetic compounds; topical antiinflammatories; and the like. The topically-active compound incorporated into the anhydrous compositions of the present invention can be soluble or insoluble in the volatile liquid carrier vehicle. However, the composition of the present invention is especially useful in the topical delivery of particulate, topically-effective compounds that that are homogeneously dispersed throughout the stable, anhydrous composition.

Accordingly, it is an object of the present invention to provide an anhydrous, topically-effective composition

including either a solubilized topically-active compound or an insoluble and suspended topically-effective compound dispersed in a suitable, volatile liquid carrier vehicle, such that the composition is stable and resists phase separation.

Another object of the present invention is to provide a stable, anhydrous, topically-effective composition including a topically-active compound, a finely-divided silica, a suspending wax composition and a suitable volatile liquid carrier vehicle.

Another object of the present invention is to provide an anhydrous, topically-effective composition that efficiently delivers a topically-effective cosmetic or medicinal compound upon application to skin or hair.

Another object of the present invention is to provide an anhydrous, topically-effective composition useful as an antiperspirant and including a volatile silicone or volatile hydrocarbon compound; an astringent salt; a finely-divided silica; and a suspending wax composition.

Still another object of the present invention is to provide an anhydrous antiperspirant composition having an unexpectedly frictionless application to the skin and including a volatile silicone or volatile hydrocarbon compound; an astringent salt; a finely-divided silica; and a suspending wax composition.

Another object of the present invention is to provide an anhydrous, topically-effective composition that is not tacky or sticky after topical application, and that dries relatively quickly after topical application.

Another object of the present invention is to provide an anhydrous, topically-effective composition that is useful as an antiperspirant and that is essentially nonwhitening and nonstaining to the skin and to clothing after topical application.

It is also a further object of the present invention to provide an anhydrous antiperspirant composition that exhibits unexpectedly smooth topical application to the skin; effectively delivers the topically-active compound; and has superior sensory properties.

Other objects of the present invention include providing an anhydrous, topically-effective composition for the administration of topically-active compounds, such as topically effective drugs and medicaments, topical anesthetics, sunscreen agents, skin-soothing emollients and other topical cosmetic compounds; topical antiinflammatories and the like by incorporating the topically-active compound in an anhydrous composition comprising a finely-divided silica, a volatile silicone or a volatile hydrocarbon and a suspending wax composition.

The above and other objects and advantages of the present invention will become more apparent from the following detailed description of the preferred embodiments.

## DETAILED DESCRIPTION OF THE INVENTION

The stable, anhydrous, topically-effectice composition of the present invention includes from 0.01 % to 30% by weight of a topically-active compound, such as an astringent salt having antiperspirant properties; from 0.1% to 15% by weight of a finely-divided silica, as a thickening agent; from 20% to 95% by weight of a volatile liquid carrier as defined above; and from 1% to 10% by weight of a suspending wax composition.

In addition, the suspending wax composition utilized in the present invention comprises from 25% to 35% by weight of a natural or synthetic wax, like castor wax; from 30% to 45% by weight of a volatile silicone or a volatile hydrocarbon; and from 25% to 40% by weight of an ester. The suspending wax composition is essential to the efficacy of the present invention and, as will be discussed more fully hereinafter, the components of the suspending wax composition must be preblended to form the suspending wax composition before admixing the suspending wax composition with the other essential ingredients of the composition the present invention. It has been found that adding the individual components of the suspending wax composition to prepare a composition of the present invention does not effectively reduce or eliminate phase separation of the composition.

In accordance with an important feature of the present invention, a wide variety of topically-active compounds can be incorporated into the stable, anhydrous compositions of the present invention. Such topically-active compositions include both cosmetic and medicinal compounds that act upon contact to the skin or hair. In accordance with another important feature of the present invention, the topically-active compound can be solubilized in the composition of the present invention or can be present as an insoluble, particulate material. In either case the anhydrous, topically-effective composition of the present invention is effective and is resistant to composition separation. In general, the topically-effective, anhydrous compositions of the present invention demonstrated essentially no phase separation if the topically-active compound is solubilized in the compositions. Furthermore, if the topically-active compound is insoluble in the composition, the anhydrous composition demonstrates essentially no phase separation if the insoluble topically-active compound has a particle size less than about 1000 μm and preferably less than about 500 μm. To achieve full advantages of the present invention, the topically-active compound has a particle size less than about 100 μm.

Therefore, the topically-active compound can be a cosmetically-active compound, a medically-active compound or any other compound that is useful upon application to the skin or hair. Such topically-active compounds include antiperspirant compounds, antidandruff compounds, antibacterial compounds, antifungal compounds, anti-inflammatory compounds, topical anesthetics, topical drugs, sunscreen compounds dermatological compounds and other cosmetic and medical topically-effective compounds.

Therefore, in accordance with an important feature of the present invention, the stable, anhydrous, topically-effective composition can include any of the generally-known antiperspirant compounds such as finely-divided solid astringent salts like aluminum chlorohydrate, aluminum chlorohydrox, zirconium chlorohydrate, and complexes of aluminum chlorohydrate with zirconyl chloride and/or hydroxychloride, either in the presence or absence of an amino acid buffer such as glycine. In general, the amount of the topically-active compound, and in particular the amount of the antiperspirant compound, i.e., aluminum zirconium tetrachlorohydrex glycinate, in the composition can range from 0.01% to 30% by weight of the total composition, and to achieve the full advantage of the present invention, is present in the range of from 5% to 20% by weight.

In addition to antiperspirant compounds, other topically-active compounds can be included in the anhydrous compositions of the present invention in a sufficient amount to perform their intended function. For example, zinc oxide, titanium dioxide or similar compounds can be included if the composition is intended to be a sunscreen. Similarly, topically-active drugs, like antifungal compounds; antibacterial compounds; anti-inflammatory compounds; topical anesthetics; skin rash, skin disease and dermatitis medications; and anti-itch and irritation-reducing compounds can be included in the compositions of the present invention. For example, analgesics such as benzocaine, dyclonine hydrochloride, aloe vera; anesthetics such as butamben picrate, lidocaine hydrochloride, xylocaine; antibacterials and antiseptics, such as povidone-iodine, polymyxin b sulfate-bacitracin, zinc-neomycin sulfate-hydrocortisone, chloramphenicol, methylbenzethonium chloride, and erythromycin and the like; antiparasitics, such as lindane; deodorants, such as chlorophyllin copper complex, aluminum chloride, aluminum chloride hexahydrate, and methylbenzethonium chloride; essentially all dermatologicals, like acne preparations, such as benzoyl peroxide, erythromycinbenzoyl peroxide, clindamycin phosphate, 5,7-dichloro-8-hydroxyquinoline ; anti-inflamatory agents, such as alclometasone dipropionate, betamethasone valerate; burn relief ointments, such as o-amino-p-toluenesulfonamide monoacetate; depigmenting agents, such as monobenzone; dermatitis relief, such as the active steroid amcinonide, diflorasone diacetate, hydrocortisone; diaper rash relief, such as methylbenzethonium chloride; emollients and moisturizers, such as mineral oil, PEG-4 dilaurate, lanolin oil, petrolatum, mineral wax; fungicides, such as butocouazole nitrate, haloprogin, clotrimazole; herpes treatment drugs, such as 9-[-(2-hydroxyethoxy) methyl] guanine; pruritic medications, such as alclometasone dipropionate, betamethasone valerate, isopropyl myristate MSD ; psoriasis, seborrhea and scabicide agents, such as anthralin, methoxsalen, coal tar; sunscreens, such as octyl p-(dimethylamino)-benzoate, octyl methoxycinnamate, oxybenzone; steroids, such as 2-(acetyloxy)-9-fluoro-1',2',3',4'-tetrahydro-11-hy- droxypregna-1,4-dieno[16,17-b]naphthalene-3,20-dione, and 21-chloro-9-fluoro-1',2',3',4'-tetrahydro-11b-hydroxypregna-1,4-dieno[16z-17-b] naphthalene-3,20-dione. Any other medication capable of administration topically also can be incorporated into the anhydrous composition of the present invention in a sufficient amount to perform its intended function.

The topically-active compound of the composition is dispersed into a non-aqueous, volatile liquid carrier, such as a volatile silicone or a volatile hydrocarbon. It should be understood that although the composition of the present invention is preferably an anydrous composition, it has been found that amounts of water up to about 20% by weight of the composition can be present without adversely affecting the composition. The water can be included intentionally or inadvertantly an ingredient in a component of the composition. However, to avoid a sticky feeling after application of the topically-effective composition to the skin, the amount of water should be present at less than 10% by weight, and to achieve the full advantage of the present invention, at less than about 5% by weight of the composition. After topical application of a composition of the present invention, the non-aqueous, volatile liquid carrier facilitates the rapid absorption of the topically-active compound into the skin, thereby eliminating the wet feeling of the carrier vehicle. The volatile liquid carrier then slowly evaporates, leaving the topically-active compound in contact with the skin.

Suitable non-aqueous volatile liquid carriers useful in the composition of present invention include the volatile, low molecular weight polydimethylsiloxane compounds. The volatile, low molecular weight polydimethylsiloxane compound can be either a linear or a cyclic polydimethylsiloxane compound, as long as the polydimethylsiloxane compound has sufficient volatility to volatilize from the skin after topical application of the composition onto the skin. Preferably the polydimethylsiloxane is a cyclic siloxane, like cyclomethicone. In general, the volatile silicones, such as cyclomethicone, feel very rich as they are applied to the skin, but then evaporate relatively quickly to leave only the non-volatile components on the skin.

Overall, volatile polydimethylsiloxane compounds useful in the compositions of the present invention include polydimethylsiloxane compounds having a viscosity in the range of from 0,005 to 0.1 $cm^2/s$, (0.5 cSt (centistokes) to 10 cSt). The preferred volatile polydimethylsiloxanes have a viscosity in the range of from 0,02 to 0,06 $cm^2/s$ (2cSt to 6 cSt).

The cyclic, low molecular weight, volatile polydimethylsiloxanes, named in the CTFA Dictionary as cyclomethicones, are the preferred siloxanes used in the composition of the present invention. To achieve the full advantage of the present invention, the cyclomethicones used in the compositions of the present invention are low viscosity, low molecular weight, water-insoluble cyclic compounds having an average of about 3 to about 6 -[O-Si($CH_3$)$_2$]-repeating group units per molecule; boil at atmospheric pressure in a range of from 150°C to 250°C; and have viscosities at 25°C of from 0.02 to 0.06 $cm^2/s$ (2 to about 6 centistokes). The polydimethyl cyclosiloxanes having an average of about 4 to about 5 repeating units per molecule, i.e., the tetramer and pentamer, are especially preferred. Suitable cyclomethicones are available commercially under the tradenames SILICONE 344 FLUID and SILICONE 345 FLUID from Dow Corning Corporation, Midland, MI, and SILICONE SF-1173 and SILICONE SF-1202 from General Electric, Waterford, NY, the tetramer being listed first in each instance.

An example of a linear, low molecular weight, volatile polydimethylsiloxane compound that is useful in the composition and method of the present invention is the compound named in the CTFA Dictionary as hexamethyldisiloxane, available commercially under the tradename DOW CORNING 200 FLUID, from Dow Corning Corp., Midland, MI. Hexamethyldisiloxane has a viscosity of 0.0065 $cm^2/s$ (0.65 cSt) is highly volatile, is non-greasy, provides lubrication for topical application of the composition of the present invention to the skin. Other linear polydimethylsiloxanes, such as decamethyltetrasiloxane, having a boiling point of about 195°C and a viscosity of 0.015 $cm^2/s$ (1.5 cSt); octamethyltrisiloxane; and dodecamethylpentasiloxane, also have sufficient volatility to be useful in the composition of the present invention. In general, it has been found that linear, low molecular weight, volatile polydimethylsiloxane compounds having a viscosity at 25°C and 1atm. (760 mm. Hg) pressure in the range of from 0.005 to 0,05 $cm^2/s$ (0.5 cSt to 5 cSt), and a boiling point at atmospheric pressure ranging from 100°C to 250°C, are preferred for use in the composition and method of the present invention.

The volatile liquid carrier, included in the composition of the present invention also can be a volatile hydrocarbon, such as a hydrocarbon including from 10 carbon atoms to 30 carbon atoms, that has sufficient volatility to slowly volatilize from the skin or hair after application of the topically-effective composition. The volatile hydrocarbons provide essentially the same benefits as the volatile silicone, such as lubrication and a rich feel during application.

The preferred volatile hydrocarbon compound is an aliphatic hydrocarbon including from 12 to 24 carbon atoms, and having a boiling point in the range of from 100°C to 300°C. Exemplary volatile hydrocarbons are depicted in general structural formula (I), wherein n ranges from 2 to 5,

```
         CH3         CH3
          |           |
H3C- (-C-CH2-)nCH-CH3                    (I)
          |
         CH3
```

Examples of volatile hydrocarbons useful in the anhydrous composition of the present invention are the commercially-available compounds PERMETHYL 99A and PERMETHYL 101A, corresponding to compounds of general structure (I) wherein n is 2 and 3, respectively, available from Permethyl Corporation, Frazer, PA. A volatile hydrocarbon compound is useful in the clear composition of the present invention either alone, in combination with another volatile hydrocarbon, or in combination with a volatile silicone.

The finely-divided silica used in the composition of the present invention acts as a suspending agent for the particulate topically-active compound in the volatile liquid carrier vehicle; aids in absorbing the volatile liquid carrier vehicle; and aids in reducing and preventing composition separation. The finely-divided silica is present in the compositions of the present invention in the range of from 0.1% to 3%, and preferably in the range of from 0.5% to 2%, by weight of the composition to provide a sufficient composition consistency suitable for use in a roll-on antiperspirant product. Amounts of finely-divided silica in excess of 3%, and up to 15%, by weight of the composition can be used to achieve a composition having a cream or paste consistency. Including amounts of finely-divided silica in excess of about 15% by weight provides a composition that has a gritty, and therefore, unpleasant and unacceptable consistency.

In accordance with an important feature of the present invention, the finely-divided silica should have a particle size in the range of from 0.001 to 0.050 μm, and preferably in the range from 0.005 to 0.030 μm. To achieve the full advantage of the present invention, the finely-divided silica has a particle size in the range of from 0.010 to 0.020 μm. A suitable finely-divided silica is fumed silica having a particle size ranging from 0.14 to 0.16 μm, and available from Cabot Corp., Tuscola, FL under the tradename CAB-O-SIL, or from Degussa Corp., Teterboro, NJ under the tradename AEROSIL COLLOIDAL SILICA.

The stable, anhydrous topically-effective compositions of the present invention also include from 1% to

10%, and preferably from 1.5% to 7%, by weight of the composition of a suspending wax composition. The suspending wax compo- sition includes from 25% to 35% by weight of a natural or a synthetic wax; from 30% to 45% by weight of a volatile silicone or a volatile hydrocarbon; and from 25% to 45% by weight of an ester. In accordance with an important feature of the present invention, the suspending wax composition must be included in a topically-effective composition of the present invention, as opposed to individually adding the three separate components of the suspending wax composition to the topically-effective composition.

The suspending wax composition is prepared, for example, by dispersing about 30 parts by weight of a wax, like castor wax, in a mixture comprising about 16.5 parts by weight of an ester like dioctyl adipate and about 18.5 parts by weight cyclomethicone. The resulting dispersion was heated to about 88°C (190°F), and a mixture comprising about 16.5 parts by weight dioctyl adipate and about 18.5 parts by weight cyclomethicone is added to the heated, fused dispersion, under continuous high-shear stirring, as a thin stream. When the addition of the thin stream was completed, the resulting mixture was shock cooled to provide a paste-like suspending wax composition useful in the composition of the present invention.

The wax used in the suspending wax composition can be a natural or synthetic wax, such as castor wax, beeswax, carnauba wax, ozokerite wax, candellila wax, hydrogenated lanolin, cocoa butter and polyethylene. The particular identity of the wax is not critical as long as the wax provides a smooth and creamy sensory feeling and can be incorporated into the paste-like suspending wax composition, as described above.

The volatile silicone or volatile hydrocarbon that can be included in the suspending wax composition are the same volatile silicones and volatile hydrocarbons that are described above and that are independently included in the anhydrous, topically-effective composition of the present invention. For example, volatile silicones that can be included in the suspending wax composition include the low molecular weight polydimethylsiloxane compounds, such as the cyclic polydimethylsiloxane tetramer and the cyclic polydimethylsiloxane pentamer, available commercially under the tradenames SILICONE 344 FLUID and SILICONE 345 LIQUID, respectively, from Dow Corning Corp, Midland, MI., and SF-1173 and SF-1202, respectively, from General Electric, Waterford, NY. A suitable volatile hydrocarbon can be substituted for, or used in conjunction with, the volatile silicone. Volatile hydrocarbons having from about 12 to about 24 carbon atoms, and a boiling point in the range of from 100°C to 300°C, such as, for example, PERMETHYL 99A available from Permethyl Corp., Frazer, PA., are suitable for use in the suspending wax composition.

An ester also is included in the suspending wax composition. Although the identity of the specific ester, or combination of esters, is not especially critical, it has been found that suitable esters include at least 10 carbon atoms, and preferably the ester includes from 12 to 32 carbon atoms. For example, suitable esters include those comprising an alcohol or a polyol including from eight to about carbon atoms and a carboxylic acid including from two to about twelve carbon atoms, or conversely, an alcohol or polyol including from two to about twelve carbon atoms with a carboxylic acid including from eight to twenty carbon atoms. Examples of suitable esters include, but are not limited to, dioctyl adipate, $C_{12}$-$C_{15}$ alcohol benzoate, laureth-3 benzoate, neopentyl glycol dioctanoate, isodecyl neopentanoate, cetyl stearate, and isocetyl stearate. It should be understood that the straight chain esters (cetyl stearate) are preferred because they are faster drying than the corresponding branched esters (isocetyl stearate).

Unexpectedly, it was found that including a sufficient amount of the suspending wax composition in an anhydrous, topically-effective composition of the present invention provides a stable composition that resists phase separation, and that eliminates the need to include organoclay suspending agents. Consequently, the topically-effective composition is suitable as a cosmetic roll-on, cream or paste product that does not require shaking prior to use, that is essentially nonwhitening, nonstaining and non-oily after topical application, that dries quickly after topical application, and that exhibits improved efficacy and sensory properties.

Optionally, one or more cosolvents may be included in a topically-effective composition of the present invention to further aid in the reduction and elimination of phase separation, whitening, and staining; and to help provide favorable sensory properties to the composition. Suitable cosolvents include a nonvolatile silicone oil, a high molecular weight polyol, an oil-soluble surfactant, a high molecular weight ester and similar organic compounds. For example, the composition of the present invention may include a compatible skin-soothing cosolvent, such as a $C_{12}$-$C_{15}$ alcohol benzoate, a dimethylsilicone fluid or a phenylsilicone fluid. The cosolvent, or combination of cosolvents, can be present in the topically-effective composition of the present invention in an amount ranging from 5% to 30%, and preferably from 10% to 30%, by weight of the composition.

The nonvolatile silicone oil cosolvent can be a nonvolatile dimethylpolysiloxane fluid or a non-volatile diphenylpolysiloxane fluid. A preferred nonvolatile silicone is a dimethylpolysiloxane fluid listed in the CTFA Dictionary as a dimethicone and has a viscosity of at least about 0,1 $cm^2/s$ (10 centistokes).

The optional incorporation of a high molecular weight polyol or an oil-soluble surfactant into a topically-effective composition of the present invention also aids in the reduction or elimination of composition phase separation, the reduction of whitening and staining after composition application and the enhancement of the

sensory properties of the composition. High molecular weight polyols or oil-soluble surfactants that can be included in a composition of the present invention include, but are not limited to, polysorbate 60, polypropylene glycol, decyl pyrrolidone, nonoxynol-2, and similar nonionic surfactants and polyols. Yet another optional cosolvent that can be included in a composition of the present invention is an ester having at least 10 carbon atoms, and preferably having between 10 and about 30 carbon atoms. Suitable esters include dioctyl adipate, laureth-3 benzoate, neopentyl glycol dioctanoate, isodecyl neopentanoate, and $C_{12}$-$C_{15}$ alcohol benzoates.

Furthermore, minor amounts of emollients such as, fatty esters, fatty alcohols, mineral oil, polyether siloxane copolymers and the like, also can be included in a topically-effective composition of the present invention. Usually, such optional emollients are included in amounts ranging from 0.1% to 10% by weight of the topically-effective composition. Examples of optional emollients include, but are not limited to, isopropyl myristate, isopropyl palmitate, cetyl acetate, cetyl propionate, diisopropyl adipate, and PPG-15 stearyl ether.

In addition to the ingredients listed above, the anhydrous, topically-effective compositions of the present invention also may include other optional ingredients that are conventionally included in topical cosmetic and medicinal compositions of this character. For example, fragrances can be incorporated into the anhydrous, topically-effective composition in an amount within the range of from 0.1% to 10% based on the total weight of the composition. The composition of the present invention, when applied to skin, fixes a substantive fragrance film on the skin that resists water but that can be removed by washing. Other optional ingredients that can be included in the anhydrous composition of the present invention include, but are not limited to, drying agents, like talc or DRY ELO® (aluminum starch octenylsuccinate); preservatives; and dyes. Generally, such optional ingredients are present in the compositions of the present invention in an amount of about 10% or less by weight. In addition, although the necessity of including an organoclay is virtually eliminated by the use of the suspending wax composition, an organo-clay may be included in a composition of the present invention as an additional suspending agent in an amount of up to 20% by weight of the composition. An exemplary organoclay is a quaternized three-layer clay exfoliated with a polar solvent, like a quaterized montmorillonite clay exfoliated with propylene glycol.

The following specific examples are illustrative of the anhydrous, topically-effective compositions of the present invention. However, it should be understood that the present invention is not limited to the specific examples set forth below. By varying the proportions and the type of each of the essential ingredients within the indicated ranges, a composition of the present invention can be prepared in liquid, cream or paste form. In the following examples, all amounts of the various ingredients are expressed by weight percentages unless otherwise specificified. Trade marks are acknowledged as such. In each of the following examples, the suspending wax composition includes about 30% castor wax, about 37% cyclomethicone, and about 33% dioctyl adipate; and is a paste-like, gel material.

EXAMPLE 1

| Ingredient | % (by weight) |
|---|---|
| Fumed silica1) | 1.5 |
| Cyclomethicone2) | 73.5 |
| Aluminum Zirconium Tetrachlorohydrex Glycinate3) | 22.0 |
| Suspending Wax Composition | 3.0 |

1) CAB-O-SIL, Cabot Corporation, Tuscola, IL

2) DOW CORNING 344 FLUID, Dow Corning Corp., Midland, MI.

3) Wickenol CPS 370, Wicken Products, Huguenot, NY

Procedure

The fumed silica was dispersed in the volatile liquid cyclomethicone, and the mixture was thoroughly blended until homogeneous. The aluminum chlorohydrate and the suspending wax composition then were added

to the silica-silicone mixture, and the resulting mixture was charged through a colloid mill for at least two minutes until homogeneous. The resulting composition had physical properties suitable for use as a roll-on antiperspirant product. When topically-applied to the skin, the antiperspirant product was dry as opposed to oily or sticky; and the antiperspirant product exhibited excellent esthetic properties. The antiperspirant product showed negligible phase separation after a 4 month aging period at 27°C.

EXAMPLE 2

| Ingredient | % (by weight) |
|---|---|
| Fumed silica 1) | 1.5 |
| Dimethicone 4) | 20.0 |
| Cyclomethicone 2) | 53.5 |
| Aluminum Zirconium Tetrachlorohydrex Glycinate 3) | 22.0 |
| Suspending Wax Composition | 3.0 |

4) SF-9650, General Electric, Waterford, N.Y.

Procedure

The fumed silica was dispersed in the dimethicone and cyclomethicone, and the mixture was thoroughly blended until homogeneous. The aluminum chlorohydrate and suspending wax composition then were added to the silica-silicone mixture, and the resulting mixture was mixed at a high shear in a colloid mill until homogeneous. The resulting composition possessed physical properties suitable for use as a roll-on antiperspirant product. When topically applied to the skin, the antiperspirant product was dry, as opposed to oily or sticky, and the antiperspirant product exhibited excellent esthetic properties. The antiperspirant product showed negligible phase separation after a 5 month aging period at 27°C.

EXAMPLE 3

| Ingredient | % (by weight) |
|---|---|
| Fumed silica 1) | 1.5 |
| $C_{12}$-$C_{15}$ Alcohol Benzoate 5) | 10.0 |
| Cyclomethicone 2) | 63.5 |
| Aluminum Zirconium Tetrachlorohydrex Glycinate 3) | 22.0 |
| Suspending Wax Composition | 3.0 |

5) FINSOLV TN, Finetex, Inc., Elmwood Park, NJ.

Procedure

The fumed silica was dispersed in the $C_{12}$-$C_{15}$ alcohol benzoate and the cyclomethicone, and the mixture was thoroughly blended until homogeneous. The aluminum chlorohydrate and the suspending wax composition then were added to the silica-silicone-benzoate mixture, and the resulting mixture was charged through a colloid mill for at least 2 minutes. The resulting composition possessed physical properties suitable for use as a roll-on antiperspirant product. When topically-applied to the skin, the resulting antiperspirant product was dry, as opposed to oily or sticky, and the antiperspirant product exhibited excellent esthetic properties. The antiperspirant product showed negligible phase separation after a 4 month aging period at 27°C.

EXAMPLE 4

| Ingredient | % (by weight) |
|---|---|
| Fumed silica[1)] | 1.50 |
| Polysorbate 60[6)] | 0.24 |
| $C_{12}$-$C_{15}$ Alcohol Benzoate[5)] | 10.00 |
| Cyclomethicone[2)] | 63.26 |
| Aluminum Zirconium Tetrachlorohydrex Glycinate[3)] | 22.0 |
| Suspending Wax Composition | 3.00 |

6) TWEEN 60, ICI Americas, Wilmington DE.

Procedure

The fumed silica was dispersed in a blend of polysorbate 60, the $C_{12}$-$C_{15}$ alcohol benzoate and the cyclomethicone, then the mixture was thoroughly blended until homogeneous. The aluminum chlorohydrate and the suspending wax composition was added to the homogeneous mixture, and the resulting mixture was charged through a colloid mill for at least 2 minutes until homogeneous. The resulting composition possessed physical properties suitable for use as a roll-on antiperspirant product. When topically-applied to the skin, the antiperspirant product was dry, as opposed to oily or sticky, and the antiperspirant product exhibited excellent esthetic properties. The antiperspirant product showed essentially no phase separation after a 4 month aging period at 27°C.

EXAMPLE 5

| Ingredient | % (by weight) |
|---|---|
| Fumed silica[1)] | 1.50 |
| Dimethicone[4)] | 15.00 |
| $C_{12}$-$C_{15}$ Alcohol Benzoate[5)] | 5.00 |
| Cyclomethicone[2)] | 53.73 |
| Polysorbate 60[6)] | 0.12 |
| Aluminum Zirconium Tetrachlorohydrex Glycinate[3)] | 22.0 |
| Suspending Wax Composition | 2.65 |

Procedure

The fumed silica, dimethicone, and $C_{12}$-$C_{15}$ alcohol benzoate were thoroughly admixed until the fumed silica was uniformly dispersed. The cyclomethicone and the polysorbate 60 were added to the mixture, and the resulting mixture then was transferred to a colloid mill and mixed for 2 minutes until the mixture was homogeneous. The zirconium chlorohydrate then was added, and the composition was mixed until the zirconium chlorohydrate was completely dispersed. The suspending wax composition then was added to the mixture, and the resulting mixture was mixed until homogeneous. The homogeneous mixture then was returned to the colloid mill and mixed for at least 3 minutes. The resulting composition possessed physical properties suitable for use as a roll-on antiperspirant product. When topically-applied to the skin, the antiperspirant product was dry, as opposed to oily or sticky, and the antiperspirant product exhibited excellent esthetic properties. The antiperspirant product showed no phase separation after a 6 month aging period at 27°C.

EXAMPLE 6

| Ingredient | % (by weight) |
|---|---|
| Cyclomethicone[2)] | 47.55 |
| Aluminum Zirconium Tetrachlorohydrex Glycinate[3)] | 22.00 |
| Fumed Silica[1)] | 1.50 |
| Suspending Wax Composition | 2.65 |
| Dimethicone[4)] | 20.00 |
| Hydrophobic Starch Derivative[7)] | 1.00 |
| Dioctyl Adipate[8)] | 3.50 |
| Fragrance | 0.30 |
| Starch/Dextrin Fragrance | 1.50 |

7) DRY FLO, National Starch and Chemical Co., Bridgewater, NJ

8) WICKENOL 158, Wicken Products, Huguenot, NY (Aluminum starch octenylsuccinate)

Procedure

The fumed silica, dimethicone, and cyclomethicone were thoroughly admixed until the fumed silica was uniformly dispersed. The mixture then was transferred to a colloid mill and mixed for at least 2 minutes, or until the mixture was homogeneous. The zirconium chlorohydrate and the hydrophobic starch derivative then were added to the mixture, and the resulting mixture was blended until both additives were completely dispersed. The suspending wax composition then was added to the mixture, and the resulting mixture was mixed until homogeneous. A premix of the dioctyl adipate and the fragrances then was added to the homogeneous mixture. The resulting mixture was returned to the colloid mill and mixed for at least 3 minutes. The final composition possessed physical properties suitable for use as a roll-on antiperspirant product. When topically-applied to the skin, the antiperspirant product was dry, as opposed to oily or sticky, and the antiperspirant product exhibited exceptional esthetic properties. The antiperspirant product showed negligible phase separation after a 6 month aging period at 27°C.

EXAMPLE 7

| Ingredient | % (by weight) |
|---|---|
| Cyclomethicone2) | 38.13 |
| Aluminum Zirconium Tetrachlorohydrex Glycinate3) | 22.00 |
| Fumed Silica1) | 0.80 |
| Suspending Wax Composition | 2.65 |
| Polysorbate 606) | 0.12 |
| Dimethicone4) | 10.00 |
| $C_{12}$-$C_{15}$ Alcohol Benzoate5) | 10.00 |
| Quaternium-18 Hectorite in cyclomethicone9) | 10.00 |
| Hydrophobic Starch Derivative7) | 1.00 |
| Dioctyl Adipate8) | 3.50 |
| Fragrance | 0.30 |
| Starch/Dextrin Fragrance | 1.50 |

9) BENTONE GEL VS/5 PC, NL Chemicals, Hightstown, NJ

Procedure

The fumed silica, dimethicone, cyclomethicone, polysorbate 60 and $C_{12}$-$C_{15}$ alcohol benzoate were thoroughly admixed until the fumed silica was uniformly dispersed. The mixture then was transferred to a colloid mill and mixed for at least 2 minutes, or until homogeneous. The zirconium chlorohydrate and the hydrophobic starch derivative then were added to the mixture, and the resulting mixture was mixed until the zirconium chlorohydrate was completely dispersed. The suspending wax composition and the BENTONE GEL then were added to the mixture, and the resulting mixture was mixed until homogeneous. A premix of the dioctyl adipate and the fragrances then was added to the homogeneous mixture. The resulting mixture was returned to the colloid mill and mixed for at least 3 minutes. The composition then was mixed with a turbine propeller until the composition was uniform and homogeneous. The composition possessed physical properties suitable for use as a roll-on antiperspirant product. When topically-applied to the skin, the antiperspirant product was dry, as opposed to oily or sticky, and the antiperspirant product exhibited exceptional esthetic properties. However, because of the presence of an organoclay, i.e., the BENTONE GEL, this particular antiperspirant product demonstrated increased whitening and staining of the skin and clothes. The antiperspirant product showed negligible phase separation after a 6 month aging period at 27°C.

EXAMPLE 8

| Ingredient | % (by weight) |
|---|---|
| Fumed silica1) | 3.2 |
| Cyclomethicone2) | 72.8 |
| Titanium Dioxide | 20.0 |
| Suspending Wax Composition | 4.0 |

Procedure

The fumed silica is dispersed in the volatile liquid cyclomethicone and the mixture is thoroughly blended until homogeneous. The titanium dioxide and the suspending wax composition then are added to the silica-silicone mixture, and the resulting mixture is charged through a colloid mill for at least two minutes until homogeneous. The resulting composition has physical properties suitable for use as a paste sunscreen product. When topically-applied to the skin, the sunscreen product is dry as opposed to oily or sticky; and the sunscreen product exhibits excellent esthetic qualities.

EXAMPLE 9

| Ingredient | % (by weight) |
|---|---|
| Fumed silica1) | 5.0 |
| Cyclomethicone2) | 90.9 |
| Benzethonium Chloride | 0.1 |
| Suspending Wax Composition | 4.0 |

Procedure

The fumed silica is dispersed in the dimethicone and volatile liquid cyclomethicone, and the mixture is thoroughly blended until homogeneous. The benzethonium chloride and suspending wax composition then are added to the silica-silicone mixture, and the resulting mixture is mixed at a high shear in a colloid mill until homogeneous. The resulting composition possesses physical properties suitable for use as a paste antibacterial product. When topically applied to the skin, the antibacterial product is dry, as opposed to oily or sticky, and the antibacterial product exhibits excellent esthetic properties.

Alternate volatile silicones, and mixtures of volatile silicones, can be substituted for the particular volatile silicone carrier vehicle used in the preceding examples. Similarly, other finely-divided silica compounds, such as AEROSIL COLLOIDAL SILICA, available from Degussa Corp., Teterboro, NJ., can be substituted for CAB-O-SIL brand of fumed silica.

The anhydrous, topically-effective compositions of the present invention, comprising a topically-active compound, like an antiperspirant compound; a finely-divided silica; and suspending wax composition; and a suitable volatile liquid carrier vehicle, exhibit unique and superior properties upon topical applications to skin or hair. The improved physical and sensory properties include ultra-dry characteristics, both as to feel and drying time; storage stability; elimination of the shaking requirement to redistribute the topically-active compound prior to use; substantially reduced whitening of the skin and clothing after topical application; and substantially reduced staining of clothing.

**Claims**

**Claims for the following Contracting States : AT, BE, CH, DE, DK, FR, GB, IT, LI, LU, NL, SE**

1. A topically-effective composition in liquid, cream or paste form for application to the skin or hair comprising:
   (a) from 0.01 % to 30 % by weight of a topically-active compound;
   (b) from 20 % to 99 % by weight of a volatile liquid carrier which has a boiling point from 150 to 250°C if it is a polydimethyl siloxane, and from 100 to 300°C if it is a hydrocarbon;
   (c) from 0.1 to 15 % by weight of a finely-divided silica;
   (d) from 0.25 to 3.5 % by weight of a wax; and
   (e) from 0.25 % to 4.0 % by weight of an ester including at least ten carbon atoms.

2. The composition of claim 1 wherein the topically-active compound is an antiperspirant compound, an antidandruff compound, an antifungal compound, an anti-inflammatory compound, a sunscreen compound, an antibacterial compound, a topical anesthetic, a topical drug, a dermatological compound, or an analgesic compound.

**3.** The composition of claim 1 wherein the volatile liquid carrier is a cyclic volatile silicone, a linear volatile silicone, a volatile hydrocarbon or a combination thereof.

**4.** The composition of claim 1 wherein the wax is a synthetic wax, a natural wax or a combination thereof.

**5.** The composition of claim 1 wherein the ester includes from 12 carbon atoms to 32 carbon atoms.

**6.** The composition of claim 1 which additionally comprises
(d) from 1 % to 10 % by weight of a suspending wax composition, wherein the suspending wax compostion comprises from 25 % to 35 % by weight of a wax; from 30 % to 45 % by weight of a volatile silicone or a volatile hydrocarbon; and from 25 % to 40 by weight of an ester including at least ten carbon atoms.

**7.** The composition of claim 6 wherein the topically-active compound is present in the range from 1% to 25 %, preferable from 5% to 20%.

**8.** The composition of claim 7 wherein the antiperspirant compound is an astringent salt selected from aluminum salts, zirconium salts, mixed aluminum zirconium salts and mixtures thereof.

**9.** The composition of claim 8, wherein the antiperspirant compound is selected from aluminum zirconium tetrachlorohydrex glycinate (a coordination complex of aluminum zirconium tetrachlorohydrate and glycine), aluminum chlorohydrate <$Al_2 (OH)_5$> $_n$x nCl, zirconium chlorohydrate (a soluble amorphous inorganic polymer formed by partial neutralization of zirconyl chloride) and aluminum chlorohydrex.

**10.** The composition of anyone of claims 6 to 9 wherein the voltile liquid carrier is present in the range from 30% to 75% by weight.

**11.** The composition of anyone of claims 1 to 10 wherein the cyclic volatile silicone has a viscosity at 25°C in the range of from 0.02 $cm^2/s$ (2 centistokes) to 0.06 $cm^2/s$ (6 centistokes) and a boiling point at 760 mm (1 atm.) in the range of from 150°C to 250°C.

**12.** The composition of claim 11 wherein the cyclic volatile silicone is a cyclomethicone (a cyclic dimethyl polysiloxane compound) of the formula

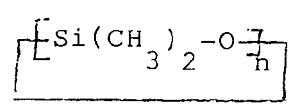

**13.** The composition of claim 12 wherein the cyclomethicone is selected from hexamethylcyclotrisiloxane; octamethylcyclotetrasiloxane; decamethylcyclopentasiloxane; dodecamethylcyclohexasiloxane; and mixtures thereof.

**14.** The composition of claim 10 wherein the linear volatile silicone has a viscosity at 25°C in the range or from 0.005 $cm^2/s$ (0.5 centistokes) to 0.05 $cm^2/s$ (5 centistokes) and a boiling point at 760 mm (1 atm.) in the range of from 100°C to 250°C.

**15.** The composition of claim 14 wherein the linear volatile silicone is selected from hexamethyldisiloxane, octamethyltrisiloxane, decamethyltetrasiloxane, dodecamethylpentasiloxane and combinations thereof.

**16.** The composition of claim 10 wherein the volatile hydrocarbon includes from 12 carbon atoms to 24 carbon atoms and has a boiling point at 760 mm (1 atm.) of from 100°C to 300°C.

**17.** The composition of claim 16 wherein the volatile hydrocarbon has the structural formula:

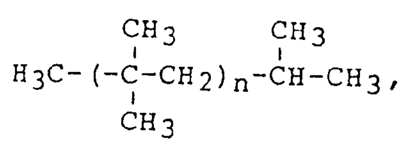

wherein n ranges from 2 to 5, and mixtures.

**18.** The composition of anyone of claims 6 to 17 wherein the finely-divided silica is present in the range of from 0.1% to 5% preferably from 0.5% to 2% by weight of the composition.

**19.** The composition of anyone of claims 6 to 18 wherein the finely divided silica has a particle size ranging from 0.001 μm to 0.050 μm, preferably from 0.010 μm to 0.020 μm.

**20.** The composition of anyone of claims 6 to 19 wherein the finely-divided silica is fumed silica.

**21.** The composition of anyone of claims 6 to 20 wherein the suspending wax composition is present in the range from 1.5% to 7% by weight of the composition.

**22.** The composition of anyone of claims 6 to 21 wherein the ester of the suspending wax composition is dioctyl adipate.

**23.** The composition of anyone of claims 6 to 22 wherein the wax of the suspending wax suspension is a synthetic wax, a natural wax, a wax-like composition, or a combination thereof, and is preferably selected from castor wax, beeswax, carnauba wax, ozokerite wax, hydrogenated lanolin, hydrogenated cocoa butter, polyethylene, and mixtures thereof.

**24.** The composition of anyone of claims 6 to 23 wherein the volatile silicone of the suspending wax composition has a viscosity in the range of from 0.005 $cm^2/s$ (0.5 centistokes) to 0.06 $cm^2/s$ (6 centistokes) and a boiling point at atmospheric pressure of from 100°C to 250°C.

**25.** The composition of anyone of claims 6 to 24 wherein the volatile hydrocarbon of the suspending wax composition is the same as the volatile hydrocarbon carrier as defined in claims 16 or 17.

**26.** The composition of anyone of claims 6 to 25 wherein the suspending wax composition comprises from 25% to 35% by weight castor wax; from 30% to 45% by weight of a volatile hydrocarbon having the structural formula:

$$H_3C-(-\underset{CH_3}{\underset{|}{\overset{CH_3}{\overset{|}{C}}}}-CH_2)_n-\overset{CH_3}{\overset{|}{C}H}-CH_3,$$

wherein n ranges from 2 to 5; and from 25% to 40% by weight of dioctyl adipate.

**27.** The composition of anyone of claims 6 to 26 further comprising from 0.1% to 30% by weight of a consolvent.

**28.** The composition of claim 27 wherein the cosolvent is a nonvolatile silicone; a benzoate ester of an alcohol having from 8 carbon atoms to 30 carbon atoms; a high molecular weight polyol; an oil-soluble surfactant; or a combination thereof.

**29.** The composition of claim 28 wherein the nonvolatile silicone is a dimethicone, a diphenylsiloxane fluid or a combination thereof, wherein the high-molecular weight polyol is preferably selected from polysorbate 60, polypropylene glycol and combinations thereof, and wherein the oil-soluble surfactant is preferable selected from decyl pyrrolidone, nonoxynol-2 and combinations thereof.

**30.** The composition of anyone of claims 6 to 28 further comprising 0.1% to 20% by weight of a quaternized three-layer clay exfoliated with a polar solvent, preferably a quaternized montmorillonite clay exfoliated with propylene glycol.

**31.** The composition of anyone of claims 6 to 30 further comprising optional ingredients selected from 0.5% to 2% by weight of a drying agent; from 3% to 10% by weight of an emollient; from 0.5% to 10% by weight of a fragrance; and combinations thereof, wherein the drying agent is preferably aluminum starch octenylsuccinate, and wherein the emollient is preferably selected from a fatty ester, a fatty alcohol, a mineral

oil, a polyether siloxane copolymer and combinations thereof.

32. A method of delivering a topically-active cosmetic compound comprising:
contacting the skin or hair with a sufficient amount of a composition as defined in anyone of claims 1 to 31.

33. A method of manufacturing a stable, topically-effective liquid, cream or paste form composition for the topical delivery of a topically-active compound comprising:
preparing a suspending wax composition comprising from 25 % to 35 % by weight of a wax; from 30 % to 45 % by weight of a volatile solvent selected from a volatile silicone, a volatile hydrocarbon, and combinations thereof, which solvent has a boiling point from 150 to 250 °C if it is polydimethyl siloxane, and from 100 to 300°C if it is a hydrocarbon; and from 25 to 40 % by weight of an ester including at least ten carbon atoms; preparing a silicone dispersion comprising from 0.1 parts to 15 parts by weight of a finely-divided silica and from 20 parts to 95 parts by weight of a volatile liquid carrier;
combining from 1 part to 10 parts by weight of the suspending wax composition with from 0.01 parts to 30 parts by weight of a topically-active compound with a silica dispersion to form a topically-active mixture; and
thoroughly admixing the topically-active mixture to form a homogeneous topically-effective composition.

**Claims fot he following Contracting States : ES, GR**

1. A process of producing a topically-effective composition in liquid, cream or paste form for application to the skin or hair comprising mixing:
(a) from 0.01 % to 30 % by weight of a topically-active compound;
(b) from 20 % to 99 % by weight of a volatile liquid carrier which has a boiling point from 150 to 250 °C if it is a polydimethyl siloxane and from 100 to 300 °C if it is a hydrocarbon;
(c) from 0.1 % to 15 by weight of a finely divided silica;
(d) from 0.25 % to 3.5 % by weight of a wax;
(e) from 0.25 to 4.0 % by weight of an ester including at least ten carbon atoms.

2. The process of claim 1 wherein the topically-active compound is an antiperspirant compound, an antidandruff compound, an antifungal compound, an anti-inflammatory compound, a sunscreen compound, an antibacterial compound, a topical anesthetic, a topical drug, a dermatological compound, or an analgesic compound.

3. The process of claim 1 wherein the volatile liquid carrier vehicle is a cyclic volatile silicone, a linear volatile silicone, a volatile hydrocarbon or a combination thereof.

4. The process of claim 1 wherein the wax is a synthetic wax, a natural wax or a combination thereof.

5. The process of claim 1 wherein the ester includes from 12 carbon atoms to 32 carbon atoms.

6. The process of claim 1 which comprises mixing components (a) to (c) as defined in claim 1 with
(d) from 1 % to 10 % of a suspending wax composition wherein the suspending wax composition comprises from 25 to 35 % by weight of a wax; from 30 % to 45 % by weight of a volatile silicone or volatile hydrocarbon; and from 25 to 40 % by weight of an ester including at least 10 carbon atoms.

7. The process of claim 6 wherein the topically-active compound is present in the range from 1% to 25 %, preferable from 5% to 20%.

8. The process of claim 7 wherein the antiperspirant compound is an astringent salt selected from aluminum salts, zirconium salts, mixed aluminum zirconium salts and mixtures thereof.

9. The process of claim 8, wherein the antiperspirant compound is selected from aluminum zirconium tetrachlorohydrex glycinate (a coordination complex of aluminum zirconium tetrachlorohydrate and glycine), aluminum chlorohydrate $<Al_2 (OH)_5>_n x\, nCl$, zirconium chlorohydrate (a soluble amorphous inorganic polymer formed by partial neutrlization of zirconyl chloride) and aluminum chlorohydrex.

**10.** The process of anyone of claims 6 to 9 wherein the volatile liquid carrier vehicle is present in the range from 30% to 75% by weight.

**11.** The process of anyone of claims 1 to 10 wherein the cyclic volatile silicone has a viscosity at 25°C in the range of from 0.02 $cm^2/s$ (2 centistokes) to 0.02 $cm^2/s$ (6 centistokes) and a boiling point at 760 mm (1 atm.) in the range of from 150°C to 250°C.

**12.** The process of claim 11 wherein the cyclic volatile silicone is a cyclomethicone (a cyclic dimethyl polysiloxane compound) of the formula

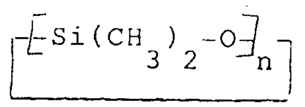

**13.** The process of claim 12 wherein the cyclomethicone is selected from hexamethylcyclotrisiloxane; octamethylcyclotetrasiloxane; decamethylcyclopentasiloxane; dodecamethylcyclohexasiloxane; and mixtures thereof.

**14.** The process of claim 10 wherein the linear volatile silicone has a viscosity at 25°C in the range of from 0.005 $cm^2/s$ (0.5 centistokes) to 0.05 $cm^2/s$ (5 centistokes) and a boiling point at 760 mm (1atm.) in the range of from 100°C to 250°C.

**15.** The process of claim 14 wherein hexamethyldisiloxane, octamethyltrisiloxane, decamethyltetrasiloxane, dodecamethylpentasiloxane and combinations thereof.

**16.** The process of claim 10 wherein the volatile hydrocarbon includes from 12 carbon atoms to 24 carbon atoms and has a boiling point at 760 mm (1atm.) of from 100°C to 300°C.

**17.** The process of claim 16 wherein the volatile hydrocarbon has the structural formula:

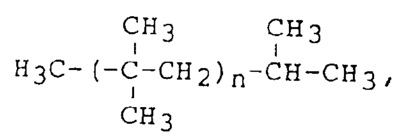

wherein n ranges from 2 to 5, and mixtures.

**18.** The process of anyone of claims 6 to 17 wherein the finely-divided silica is present in the range of from 0.1% to 5% preferably from 0.5% to 2% by weight of the composition.

**19.** The process of anyone of claims 6 to 18 wherein the finely divided silica has a particle size ranging from 0.001 μm to 0.050 μm, preferably rom 0.010 μm to 0.020 μm.

**20.** The process of anyone of claims 6 to 19 wherein the finely-divided silica is fumed silica.

**21.** The process of anyone of claims 6 to 20 wherein the suspending wax composition is present in the range from 1.5% to 7% by weight of the composition.

**22.** The process of anyone of claims 6 to 21 wherein the ester of the suspending wax composition is dioctyl adipate.

**23.** The process of anyone of claims 6 to 22 wherein the wax of the suspending wax suspension is a synthetic wax, a natural wax, a wax-like composition, or a combination thereof, and is preferably selected from castor wax, beeswax, carnauba wax, ozokerite wax, hydrogenated lanolin, hydrogenated cocoa butter, polyethylene, and mixtures thereof.

**24.** The process of anyone of claims 6 to 23 wherein the volatile silicone of the suspending wax composition has a viscosity in the range of from 0.005 $cm^2/s$ (0.5 centistokes) to 0.06 $cm^2/s$ (6 centistokes) and a boiling

point of 25°C at atmospheric pressure of from 100°C to 250°C.

**25.** The process of anyone of claims 6 to 24 wherein the volatile hydrocarbon of the suspending wax composition is the same as the volatile hydrocarbon carrier as defined in claims 16 to 17.

**26.** The process of anyone of claims 6 to 25 wherein the suspending wax composition comprises from 25% to 35% by weight castor wax; from 30% to 45% by weight of a volatile hydrocarbon having the structural formula:

$$\begin{array}{l} \quad\quad\quad CH_3 \quad\quad\quad\quad CH_3 \\ \quad\quad\quad\ | \quad\quad\quad\quad\quad\ \ | \\ H_3C-(-C-CH_2)_n-CH-CH_3, \\ \quad\quad\quad\ | \\ \quad\quad\quad CH_3 \end{array}$$

wherein n ranges from 2 to 5; and from 25% to 40% by weight of dioctyl adipate.

**27.** The process of anyone of claims 6 to 26 further comprising from about 0.1% to about 30% by weight of a cosolvent.

**28.** The process of claim 27 wherein the cosolvent is a nonvolatile silicone; a benzoate ester of an alcohol having from 8 carbon atoms to 30 carbon atoms; a high molecular weight polyol; an oil-soluble surfactant; or a combination thereof.

**29.** The process of claim 28 wherein the nonvolatile silicone is a dimethicone, a diphenylsiloxane fluid or a combination thereof, wherein the high-molecular weight polyol is preferably selected from polysorbate 60, polypropylene glycol and combinations thereof, and wherein the oil-soluble surfactant is preferable selected from decyl pyrrolidone, nonoxynol-2- and combinations thereof.

**30.** The process of anyone of claims 6 to 28 further comprising admixing 0.1% to 20% by weight of a quaternized three-layer clay exfoliated with a polar solvent, preferably a quaternized montmorillonite clay exfoliated with propylene glycol.

**31.** The process of anyone of claims 6 to 30 further comprising admixing optional ingredients selected from 0.5% to 2% by weight of a drying agent; from 3% to 10% by weight of an emollient; from 0.5% to 10% by weight of a fragrance; and combinations thereof, wherein the drying agent is preferably aluminum starch octenylsuccinate, and wherein the emollient is preferably selected from a fatty ester, a fatty alcohol, a mineral oil, a polyether siloxane copolymer and combinations thereof.

**32.** A method of delivering a topically-active cosmetic compound comprising:
contacting the skin or hair with a sufficient amount of a composition obtainable by a process as defined in anyone of claims 1 to 31.

**33.** A method of manufacturing a stable, topically-effective composition in liquid, cream or paste from for the topical d Delivery of a topically active compound comprising:
preparing a suspending wax composition comprising from 25 % to 35 % by weight of a wax; from 30 % to 45 % by weight of a volatile solvent selected from a volatile silicone, a volatile hydrocarbon, and combinations thereof, which solvent has a boiling point from 150 to 250 °C if it is polydimethylsiloxane, and from 100 to 300°C if it is a hydrocarbon; and from 25 to 40 % by weight of an ester including at least ten carbon atoms;
preparing a silicone dispersion comprising from 0.1 parts to 15 parts by weight of a finely-divided silica and from 20 to 95 parts by weight of a volatile liquid carrier;
combining from 1 part to 10 parts by weight of the suspending wax composition with from 0.01 parts to 30 parts by weight of a topically-active compound with a silica dispersion to form a topically-active mixture; and
thoroughly admixing the topically-active mixture to form a homogeneous topically-effective composition.

## Patentansprüche

### Patentansprüche für folgende Vertragsstaaten : AT, BE, CH, DE, DK, FR, GB, IT, LI, LU, NL, SE

1. Topisch wirksame Zusammensetzung in flüssiger, Creme- oder Pastenform zum Aufbringen auf die Haut oder das Haar, enthaltend:
   (a) 0.01 bis 30 Gew.-% einer topisch wirksamen Verbindung; (b) 20 bis 99 Gew.-% eines flüchtigen flüssigen Trägers mit einem Siedepunkt von 150 bis 250°C, wenn er ein polydimethylsiloxan ist, und von 100 bis 300 °C, wenn er ein Kohlenwasserstoff ist;
   (c) 0,1 bis 15 Gew.-% einer feinteiligen Kieselsäure;
   (d) 0,25 bis 3,0 Gew.- eines Wachses; und
   (e) 0,25 bis 4,0 Gew.-% eines Esters, der mindestens 10 Kohlenstoffatome enthält.

2. Zusammensetzung nach Anspruch 1, worin die topisch wirksame Verbindung ein Antiperspirans, eine Antischuppenverbindung, eine Antifungusverbindung, eine entzündungshemmende Verbindung, ein Sonnenschutzmittel, eine antibakterielle Verbindung, ein topisches Anästhetikum, eine topische Droge, eine dermatologische Verbindung oder ein Analgetikum darstellt.

3. Zusammensetzung nach Anspruch 1, worin der flüchtige flüssige Träger ein cyclisches flüchtiges Silikon, ein lineares flüchtiges Silikon, einen flüchtigen Kohlenwasserstoff oder eine Kombination dieser Substanzen darstellt.

4. Zusammensetzung nach Anspruch 1, worin das Wachs ein synthetisches Wachs, ein natürliches Wachs oder eine Kombination dieser Substanzen darstellt.

5. Zusammmensetzung nach Anspruch 1, worin der Ester 12 bis 32 Kohlenstoffatome enthält.

6. Zusammensetzung nach Anspruch 1, welche zusätzlich enthält: (d) 1 bis 10 Gew.-% einer suspendierenden Wachszusammensetzung, worin die suspendierende Wachszuammensetzung 25 bis 35 Gew.-% eines Wachses; 30 bis 45 Gew.-% eines flüchtigen Silikons oder eines flüchtigen Kohlenwasserstoffs; und 25 bis 40 Gew.-% eines Esters mit mindestens 10 Kohlenstoffatomen enthält.

7. Zusammensetzung nach Anspruch 6, worin die topisch aktive Verbindung im Bereich von 1 bis 25 %, vorzugsweise von 5 bis 20 %, vorliegt.

8. Zusammensetzung nach Anspruch 7, worin das Antiperspirans ein adstringierendes Salz, ausgewählt aus Aluminiumsalzen, Zirkonsalzen, Aluminium-Zirkon-Mischsalzen und deren Gemischen darstellt.

9. Zusammensetzung nach Anspruch 8, worin das Antiperspirans aus Aluminium-Zirkon-Tetrachlorohydrex-Glycinat (ein Koordinationskomplex aus Aluminium-Zirkon-Tetrachlorhydrat und Glycin), Aluminiumchlorhydrat $[Al_2(OH)_5]_n$x n Cl, Zirkonchlorhydrat (ein lösliches amorphes anorganisches Polymer, das durch teilweise Neutralisation von Zirkonylchlorid gebildet wird) und Aluminiumchlorohydrex ausgewählt ist.

10. Zusammensetzung nach einem der Ansprüche 6 bis 9, worin der flüchtige flüssige Träger im Bereich von 30 bis 75 Gew.-% vorhanden ist.

11. Zusammensetzung nach einem der Ansprüche 1 bis 10, worin das cyclische flüchtige Silikon eine Viskosität bei 25°C im Bereich von 0,02 $cm^2$/s (2 Centistokes) bis 0,06 $cm^2$/s (6 Centistokes) und einen Siedepunkt bei 760mm (1 atm) im Bereich von 150 bis 250°C hat.

12. Zusammensetzung nach Anspruch 11, worin das cyclische flüchtige Silicon ein Cyclomethicon (eine cyclische DimethylPolysiloxanverbindung) der Formel

$$\left[Si(CH_3)_2-O_n\right]$$

darstellt.

13. Zusammensetzung nach Anspruch 12, worin das Cyclomethicon aus Hexamethylcyclotrisiloxan;

Octamethylcyclotetrasiloxan; Decamethylcyclopentasiloxan; Dodecamethylcyclohexasiloxan; und Gemischen davon ausgewählt ist.

**14.** Zusammensetzung nach Anspruch 10, worin das lineare flüchtige Silicon eine Viskosität bei 25°C im Bereich von 0,005 $cm^2/s$ (0,5 Centistokes) bis 0,05 $cm^2/s$ (5 Centistokes) und einem Siedepunkt bei 760 mm (1 atm) im Bereich von 100 bis 250°C hat.

**15.** Zusammensetzung nach Anspruch 14, worin das lineare flüchtige Silicon aus Hexamethyldisiloxan, Octamethyltrisiloxan, Decamethyltetrasiloxan, Dodecamethylpentasiloxan und Kombinationen davon ausgewählt ist.

**16.** Zusammensetzung nach Anspruch 10, worin der flüchtige Kohlenwasserstoff 12 bis 24 Kohlenstoffatome enthält und einen Siedepunkt bei 760mm (1 atm) von 100 bis 300'C hat.

**17.** Zusammensetzung nach Anspruch 16, worin der flüchtige Kohlenwasserstoff die Strukturformel

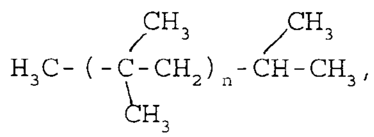

hat, worin n = 2 bis 5 bedeutet, sowie Gemische davon.

**18.** Zusammensetzung nach einem der Ansprüche 6 bis 17, worin die feinteilige Kieselsäure im Bereich von 0,1 bis 5%, vorzugsweise von 0,5 bis 2 Gew.-% der Zusammensetzung vorhanden ist.

**19.** Zusammensetzung nach einem der Ansprüche 6 bis 18, worin die feinteilige Kieselsäure eine Teilchengröße im Bereich von 0,001µm bis 0,050µm, vorzugsweise von 0,010µm bis 0,020µm hat.

**20.** Zusammensetzung nach einem der Ansprüche 6 bis 19, worin die feinteilige Kieselsäure pyrogene Kieselsäure darstellt.

**21.** Zusammensetzung nach einem der Ansprüche 6 bis 20, worin die suspendierende Wachszusammensetzung in einem Bereich von 1,5 bis 7 Gew.-% der Zusammensetzung vorhanden ist.

**22.** Zusammensetzung nach einem der Ansprüche 6 bis 21, worin der Ester der suspendierenden Wachszusammensetzung Dioctyladipat ist.

**23.** Zusammensetzung nach einem der Ansprüche 6 bis 22, worin das Wachs in der suspendierenden Wachszusammensetzung ein synthetisches Wachs, ein natürliches Wachs, eine wachsähnliche Zusammensetzung oder eine Kombination hiervon darstellt und vorzugsweise aus Ricinuswachs, Bienenwachs, Carnaubawachs, Ozokeritwachs, hydriertem Lanolin, hydrierter Kokosbutter, Polyethylen oder Gemischen davon ausgewählt ist.

**24.** Zusammensetzung nach einem der Ansprüche 6 bis 23, worin das flüchtige Silikon der suspendierenden Wachszusammensetzung eine Viskosität im Bereich von 0,005 $cm^2/s$ (0,5 Centistokes) bis 0,06 $cm^2/s$ (6 Centistokes) und einen Siedepunkt bei Atmosphärendruck von 110 bis 250°C hat.

**25.** Zusammensetzung nach einem der Ansprüche 6 bis 24, worin der flüchtige Kohlenwasserstoff in der suspendierenden Wachszusammensetzung der gleiche ist wie der flüchtige Kohlenwasserstoffträger, wie er in Anspruch 16 oder 17 definiert ist.

**26.** Zusammensetzung nach einem der Ansprüche 6 bis 25, worin die suspendierende Wachszusammensetzung 25 bis 35 Gew -% Ricinuswachs; 30 bis 45 Gew.-% eines flüchtigen Kohlenwasserstoffs mit der Strukturformel

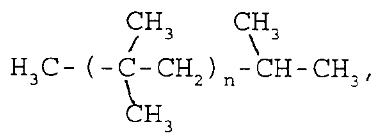

worin n = 2 bis 5 bedeutet; und 25 bis 40 Gew.-% Dioctyladipat enthält.

**27.** Zusammensetzung nach einem der Ansprüche 6 bis 26, weiterhin enthaltend 0,1 bis 30 Gew.-% eines Co-Lösungsmittels.

**28.** Zusammensetzung nach Anspruch 27, worin das Co-Lösungsmittel ein nicht-flüchtiges Silikon; einen Benzoatester eines Alkohols mit 8 bis 30 Kohlenstoffatomen; einen hochmolekularen Polyol, ein öllösliches Tensid; oder eine Kombination dieser Substanzen darstellt.

**29.** Zusammensetzung nach Anspruch 28, worin das nichtflüchtige Silicon ein Dimethicon, ein flüssiges Diphenylsiloxan oder eine Kombination dieser Substanzen, darstellt, wobei der hochmolekulare Polyol vorzugsweise aus Polysorbat 60, Polypropyleneglykol und Kombinationen hiervon ausgewählt ist, und worin das öllösliche Tensid vorzugsweise aus Decylpyrrolidon, Nonoxynol-2 und Kombinationen hiervon ausgewählt ist.

**30.** Zusammensetzung nach einem der Ansprüche 6 bis 28, weiterhin enthaltend 0, 1 bis 20 Gew.-% eines quaternisierten Dreischichttons, der mit Hilfe eines polaren Lösungsmittels aufgelockert ist, vorzugsweise ein quaternisierter Montmorillonitton, der mit Propylenglykol aufgelockert ist.

**31.** Zusammensetzung nach einem der Ansprüche 6 bis 30, weiterhin enthaltend Fakultativbestandteile, die aus 0,5 bis 2 Gew.-% eines Trockenmittels; 3 bis 10 Gew.-% eines Weichmachers; 0,5 bis 10 Gew.-% eines Duftstoffes; und Kombinationen hiervon ausgewählt sind, worin das Trockenmittel vorzugsweise Aluminium-Stärke-Octenylsuccinat darstellt, und worin der Weichmacher vorzugsweise aus Fettsäureestern, Fettalkoholen, Mineralölen, Polyethersiloxan-Copolymeren und Kombinationen dieser Substanzen ausgewählt ist.

**32.** Verfahren zum Aufbringen einer topisch wirksamen kosmetischen Zusammensetzung, welches (folgende Schritte) umfaßt:
Inberührungbringen der Haut oder des Haares mit einer ausreichenden Menge einer Zusammensetzung, wie sie in einem der Ansprüche 1 bis 31 definiert ist.

**33.** Verfahren zur Herstellung einer stabilen, topisch wirksamen flüssigen, creme- oder pastenförmigen Zusammensetzung für die topische Abgabe einer topisch wirksamen Verbindung, welches (folgende Schritte) umfaßt:
Herstellung einer suspendierenden Wachszusammensetzung, enthaltend 25 bis 35 Gew.-% eines Wachses; 30 bis 45 Gew.-% eines flüchtigen Lösungsmittels, ausgewählt aus einem flüchtigen Silikon, einem flüchtigen Kohlenwasserstoff und Kombinationen hiervon, wobei das Lösungsmittel einen Siedepunkt von 150 bis 250°C hat, wenn es ein Polydimethylsiloxan ist, und von 100 bis 300°C hat, wenn es ein Kohlenwasserstoff ist; und 25 bis 40 Gew.-% eines Esters mit mindestens 10 Kohlenstoffatomen; Herstellung einer Silikondispersion, enthaltend 0,1 bis 15 Gew.-Teile einer feinteiligen Kieselsäure und 20 bis 95 Gew.-Teile eines flüchtigen flüssigen Trägers;
Vereinigung von 1 bis 10 Gew.-Teilen der suspendierenden Wachszusammensetzung mit 0,01 bis 30 Gew.-Teilen einer topisch wirksamen Verbindung mit einer Kieselsäuredispersion zur Bildung einer topisch wirksamen Mischung; und
gründliches Vermischen der topisch wirksamen Mischung zur Bildung einer homogenen, topisch wirksamen Zusammensetzung.

**Patentansprüche für folgende Vertragsstaaten : ES, GR**

**1.** Verfahren zur Herstellung einer topisch wirksamen Zusamensetzung in flüssiger, Creme- oder Pastenform zum Aufbringen auf die Haut oder das Haar, enthaltend:
(a) 0.01 bis 30 Gew.-% einer topisch wirksamen Verbindung;
(b) 20 bis 99 Gew.-% eines flüchtigen flüssigen Trägers, der einen Siedepunkt von 150 bis 250°C hat,

wenn er ein Polydimethylsiloxan ist, und von 100 bis 300 °C hat, wenn er ein Kohlenwasserstoff ist;
(c) 0,1 bis 15 Gew.-% einer feinteiligen Kieselsäure;
(d) 0,25 bis 3,0 Gew.-% eines Wachses; und
(e) 0,25 bis 4,0 Gew.-% eines Esters, der mindestens 10 Kohlenstoffatome enthält.

2. Verfahren nach Anspruch 1, worin die topisch wirksame Verbindung ein Antiperspirans, eine Antischuppenverbindung, eine Antifungusverbindung, eine entzündungshemmende Verbindung, ein Sonnenschutzmittel, eine antibakterielle Verbindung, ein topisches Anästhetikum, eine topische Droge, eine dermatologische Verbindung oder ein Analgetikum darstellt.

3. Verfahren nach Anspruch 1, worin der flüchtige flüssige Träger ein cyclisches flüchtiges Silikon, ein lineares flüchtiges Silikon, einen flüchtigen Kohlenwasserstoff oder eine Kombination dieser Substanzen darstellt.

4. Verfahren nach Anspruch 1, worin das Wachs ein synthetisches Wachs, ein natürliches Wachs oder eine Kombination dieser Substanzen darstellt.

5. Verfahren nach Anspruch 1, worin der Ester 12 bis 32 Kohlenstoffatome enthält.

6. Verfahren nach Anspruch 1, worin die Komponenten (a) bis (c), wie sie in Anspruch 1 definiert sind, vermischt werden mit:
(d) 1 bis 10 Gew.-% einer suspendierenden Wachszusammensetzung, worin die suspendierende Wachszuammensetzung 25 bis 35 Gew.-% eines Wachses; 30 bis 45 Gew.-% eines flüchtigen Silikons oder eines flüchtigen Kohlenwasserstoffs; und 25 bis 40 Gew.-% eines Esters mit mindestens 10 Kohlenstoffatomen enthält.

7. Verfahren nach Anspruch 6, worin die topisch aktive Verbindung im Bereich von 1 bis 25 %, vorzugsweise von 5 bis 20 %, vorliegt.

8. Verfahren nach Anspruch 7, worin das Antiperspirans ein adstringierendes Salz, ausgewählt aus Aluminiumsalzen, Zirkonsalzen, Aluminium-Zirkon-Mischsalzen und deren Gemischen darstellt.

9. Verfahren nach Anspruch 8, worin das Antiperspirans aus Aluminium-Zirkon-Tetrachlorohydrex-Glycinat (ein Koordinationskomplex aus Aluminium-Zirkon-Tetrachlorhydrat und Glycin), Aluminiumchlorhydrat $[Al_2(OH)_5]_n$ x n Cl, Zirkonchlorhydrat (ein lösliches amorphes anorganisches Polymer, das durch teilweise Neutralisation von Zirkonlychlorid gebildet wird) und Aluminiumchlorohydrex ausgewählt ist.

10. Verfahren nach einem der Ansprüche 6 bis 9, worin der flüchtige flüssige Träger im Bereich von 30 bis 75 Gew.-% vorhanden ist.

11. Verfahren nach einem der Ansprüche 1 bis 10, worin das cyclische flüchtige Silikon eine Viskosität bei 25°C im Bereich von 0,02 $cm^2/s$ (2 Centistokes) bis 0,06 $cm^2/s$ (6 Centistokes) und einen Siedepunkt bei 760mm (1 atm) im Bereich von 150 bis 250°C hat.

12. Verfahren nach Anspruch 11, worin das cyclische flüchtige Silicon ein Cyclomethicon (eine cyclische Dimethyl-Polysiloxanverbindung) der Formel

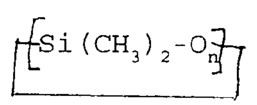

darstellt.

13. Verfahren nach Anspruch 12, worin das Cyclomethicon aus Hexamethylcyclotrisiloxan; Octamethylcyclotetrasiloxan; Decamethylcyclopentasiloxan; Dodecamethylcyclohexasiloxan; und Gemischen davon ausgewählt ist.

14. Verfahren nach Anspruch 10, worin das lineare flüchtige Silicon eine Viskosität bei 25°C im Bereich von 0,005 $cm^2/s$ (O,5 Centistokes) bis 0,05 $cm^2/s$ (5 Centistokes) und einem Siedepunkt bei 760 mm (1 atm) im Bereich von 100 bis 250°C hat.

15. Verfahren nach Anspruch 14, worin das lineare flüchtige Silicon aus Hexamethyldisiloxan, Octamethyltrisiloxan, Decamethyltetrasiloxan, Dodecamethylpentasiloxan und Kombinationen davon ausgewählt ist.

16. Verfahren nach Anspruch 10, worin der flüchtige Kohlenwasserstoff 12 bis 24 Kohlenstoffatome enthält und einen Siedepunkt bei 760mm (1 atm) von 100 bis 300°C hat.

17. Verfahren nach Anspruch 16, worin der flüchtige Kohlenwasserstoff die Strukturformel

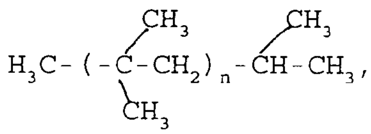

hat, worin n = 2 bis 5 bedeutet, sowie Gemische davon.

18. Verfahren nach einem der Ansprüche 6 bis 17, worin die feinteilige Kieselsäure im Bereich von 0,1 bis 5%, vorzugsweise von 0,5 bis 2 Gew.-% der Zusammensetzung vorhanden ist.

19. Verfahren nach einem der Ansprüche 6 bis 18, worin die feinteilige Kiesesäure eine Teilchengröße im Bereich von 0,001µm bis 0,050µm, vorzugsweise von 0,010µm bis 0,020µm hat.

20. Verfahren nach einem der Ansprüche 6 bis 19, worin die feinteilige Kieselsäure pyrogene Kieselsäure darstellt.

21. Verfahren nach einem der Ansprüche 6 bis 20, worin die suspendierende Wachszusammensetzung in einem Bereich von 1,5 bis 7 Gew.-% der Zusammensetzung vorhanden ist.

22. Verfahren nach einem der Ansprüche 6 bis 21, worin der Ester der suspendierenden Wachszusammensetzung Dioctyladipat ist.

23. Verfahren nach einem der Ansprüche 6 bis 22, worin das Wachs in der suspendierenden Wachszusammensetzung ein synthetisches Wachs, ein natürliches Wachs, eine wachsähnliche Zusammensetzung oder eine Kombination hiervon darstellt und vorzugsweise aus Ricinuswachs, Bienenwachs, Carnaubawachs, Ozokeritwachs, hydriertem Lanolin, hydrierter Kokosbutter, Polyethylen oder Gemischen davon ausgewählt ist.

24. Verfahren nach einem der Ansprüche 6 bis 23, worin das flüchtige Silikon der suspendierenden Wachszusammensetzung eine Viskosität im Bereich von 0,005 $cm^2/s$ (0,5 Centistokes) bis 0,06 $cm^2/s$ (6 Centistokes) und einen Siedepunkt bei Atmosphärendruck von 110 bis 250°C hat.

25. Verfahren nach einem der Ansprüche 6 bis 24, worin der flüchtige Kohlenwasserstoff in der suspendierenden Wachszusammensetzung der gleiche ist wie der flüchtige Kohlenwasserstoffträger, wie er in Anspruch 16 oder 17 definiert ist.

26. Verfahren nach einem der Ansprüche 6 bis 25, worin die suspendierende Wachszusammensetzung 25 bis 35 Gew.-% Ricinuswachs; 30 bis 45 Gew.-% eines flüchtigen Kohlenwasserstoffs mit der Strukturformel

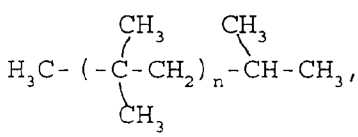

worin n = 2 bis 5 bedeutet; und 25 bis 40 Gew.-% Dioctyladipat enthält.

27. Verfahren nach einem der Ansprüche 6 bis 26, weiterhin enthaltend 0,1 bis 30 Gew.-% eines Co-Lösungsmittels.

28. Verfahren nach Anspruch 27, worin das Co-Lösungsmittel ein nicht-flüchtiges Silikon; einen Benzoatester eines Alkohols mit 8 bis 30 Kohlenstoffatomen; einen hochmolekularen Polyol, ein öllösliches Tensid; oder eine Kombination dieser Substanzen darstellt.

29. Verfahren nach Anspruch 28, worin das nicht-flüchtige Silicon ein Dimethicon, ein flüssiges Diphenylsiloxan oder eine Kombination dieser Substanzen, darstellt, wobei der hochmolekulare Polyol vorzugsweise aus Polysorbat 60, Polypropyleneglykol und Kombinationen hiervon ausgewählt ist, und worin das öllösliche Tensid vorzugsweise aus Decylpyrrolidon, Nonoxynol-2 und Kombinationen hiervon ausgewählt ist.

30. Verfahren nach einem der Ansprüche 6 bis 28, weiterhin enthaltend 0, 1 bis 20 Gew.-% eines quaternisierten Dreischichttons, der mit Hilfe eines polaren Lösungsmittels aufgelockert ist, vorzugsweise ein quaternisierter Montmorillonitton, der mit Propylenglykol aufgelockert ist.

31. Verfahren nach einem der Ansprüche 6 bis 30, weiterhin enthaltend Fakultativbestandteile, die aus 0,5 bis 2 Gew.-% eines Trockenmittels; 3 bis 10 Gew.-% eines Weichmachers; 0,5 bis 10 Gew.-% eines Duftstoffes; und Kombinationen hiervon ausgewählt sind, worin das Trockenmittel vorzugsweise Aluminium-Stärke-Octenylsuccinat darstellt, und worin der Weichmacher vorzugsweise aus Fettsäureestern, Fettalkoholen, Mineralölen, Polyethersiloxan-Copolymeren und Kombinationen dieser Substanzen ausgewählt ist.

32. Verfahren zum Aufbringen einer topisch wirksamen kosmetischen Zusammensetzung, welches (folgende Schritte) umfaßt:
Inberührungbringen der Haut oder des Haares mit einer ausreichenden Menge einer Zusammensetzung, wie sie in einem der Ansprüche 1 bis 31 definiert ist.

33. Verfahren zur Herstellung einer stabilen, topisch wirksamen flüssigen, creme- oder pastenförmigen Zusammensetzung für die topische Abgabe einer topisch wirksamen Verbindung, welches (folgende Schritte) umfaßt:
Herstellung einer suspendierenden Wachszusammensetzung, enthaltend 25 bis 35 Gew.-% eines Wachses; 30 bis 45 Gew.-% eines flüchtigen Lösungsmittels, ausgewählt aus einem flüchtigen Silikon, einem flüchtigen Kohlenwasserstoff und Kombinationen hiervon, wobei das Lösungsmittel einen Siedepunkt von 150 bis 250°C hat, wenn es ein Polydimethyl-siloxan ist, und von 100 bis 300°C hat, wenn es ein Kohlenwasserstoff ist; und 25 bis 40 Gew.-% eines Esters mit mindestens 10 Kohlenstoffatomen; Herstellung einer Silikondispersion, enthaltend 0,1 bis 15 Gew.-Teile einer feinteiligen Kieselsäure und 20 bis 95 Gew.-Teile eines flüchtigen flüssigen Trägers;
Vereinigung von 1 bis 10 Gew.-Teilen der suspendierenden Wachszusammensetzung mit 0,01 bis 30 Gew.-Teilen einer topisch wirksamen Verbindung mit einer Kieselsäuredispersion zur Bildung einer topisch wirksamen Mischung; und
gründliches Vermischen der topisch wirksamen Mischung zur Bildung einer homogenen, topisch wirksamen Zusammensetzung.

## Revendications

### Revendications pour les Etats contractants suivants : AT, BE, CH, DE, DK, FR, GB, IT, LI, LU, NL, SE

1. Composition localement efficace sous forme d'un liquide, d'une crème ou d'une pâte pour application à la peau ou aux cheveux, qui comprend :
(a) de 0,01 à 30% en poids d'un composé locale- ment actif ;
(b) de 20 à 99% en poids d'un véhicule liquide volatil dont le point d'ébullition est compris entre 150 et 250°C s'il est un polydiméthylsiloxane et entre 100 et 300°C s'il est un hydrocarbure ;
(c) de 0,1 à 15% en poids d'une silice finement divisée ;
(d) de 0,25 à 3,5% en poids d'une cire ;
(e) de 0,25 à 4,0% en poids d'un ester contenant au moins 10 atomes de carbone.

2. Composition selon la revendication 1, dans laquelle le composé localement actif est un composé antiperspirant, un composé antipelliculaire, un composé anti-fongique, un composé anti-inflammatoire, un composé filtre solaire, un composé anti-bactérien, un composé anesthésique local, un médicament local,

un composé dermatologique ou un composé analgésique.

3. Composition selon la revendication 1, dans laquelle le véhicule liquide volatil est une silicone volatile cyclique, une silicone volatile linéaire, un hydrocarbure volatil ou une combustion de ceux-ci.

4. Composition selon la revendication 1, dans laquelle la cire est une cire synthétique, une cire na- turelle ou une combinaison de celles-ci.

5. Composition selon la revendication 1, dans laquelle l'ester contient de 12 à 32 atomes de carbone.

6. Composition selon la revendication 1, qui comprend en outre :
(d) de 1 à 10% en poids d'une composition de cire de suspension dans laquelle la composition de cire de suspension comprend de 25 à 35% en poids d'une cire ; de 30 à 45% en poids d'une silicone volatile ou d'un hydrocarbure volatil ; et de 25 à 40% en poids d'un ester contenant au moins 10 atomes de carbone.

7. Composition selon la revendication 6, dans laquelle le composé localement actif est présent à raison de 1 à 25%, de préférence de 5 à 20%.

8. Composition selon la revendication 7, dans laquelle le composé antiperspirant est un sel astringent choisi parmi les sels d'aluminium, les sels de zirconium, les sels mélangés d'aluminium et de zirconium et leurs mélanges.

9. Composition selon la revendication 8, dans laquelle le composé antiperspirant est choisi parmi le tetrachlorhydrex-qlycinate de zirconium et aluminium (complexe de coordination de tetrachlorhydrate d'aluminium et zirconium et de glycine), le chlorhydrate d'aluminium $(Al_2(OH)_5)_n$ x nCl, le chlorhydrate de zirconium (polymère minéral amorphe soluble formé par neutralisation partielle de chlorure de zirconyle) et le chlorhydrex d'aluminium.

10. Composition selon l'une quelconque des revendications 6 à 9, dans laquelle le véhicule liquide volatil est présent à raison de 30 à 75% en poids.

11. Composition selon l'une quelconque des revendications 1 à 10, dans laquelle la silicone volatile cyclique présente une viscosité à 25°C comprise entre 0,02 $cm^2/s$ (2 cs) et 0,06 $cm^2/s$ (6 cs) et un point d'ébullition à 25°C et 760 mmHg (1 atm) compris entre 150 et 250°C.

12. Composition selon la revendication 11, dans laquelle la silicone volatile cyclique est une cyclométhicone (composé cyclique de diméthylpolysiloxane) de formule :

$$\left[ -(Si(CH_3)_2-O)_n- \right]$$

13. Composition selon la revendication 12, dans laquelle la cyclométhicone est choisie parmi l'hexaméthylcyclotrisiloxane ; l'octaméthylcyclotetrasiloxane ; le decaméthylcyclopentasiloxane ; le dodecaméthylcyclohexasiloxane et leurs mélanges.

14. Composition selon la revendication 10, dans laquelle la silicone volatile linéaire présente une viscosité à 25°C comprise entre 0,005 $cm^2/s$ (0,5 cs) et 0,05 $cm^2/s$ (5 cs) et un point d'ébullition à 25°C sous 760 mmHg (1 atm) de 100 à 250°C.

15. Composition selon la revendication 14, dans laquelle la silicone volatile est choisie parmi l'hexaméthyldisiloxane, l'octaméthyltrisiloxane, le decaméthyltetrasiloxane, le dodecaméthylpentasiloxane et leurs combinaisons.

16. Composition selon la revendication 10, dans laquelle l'hydrocarbure volatil comprend de 12 à 24 atomes de carbone et son point d'ébullition à 25°C et 760 mmHg (1 atm) est de 100 à 300°C.

17. Composition selon la revendication 16, dans laquelle l'hydrocarbure volatil répond à la formule structurale :

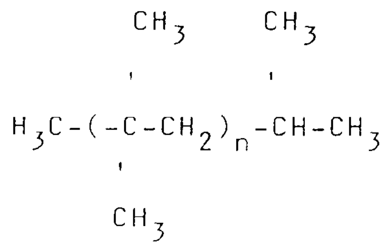

dans laquelle n est compris entre 2 et 5, et leurs mélanges.

**18.** Composition selon l'une quelconque des revendications 6 à 17, dans laquelle la silice finement divisée est présente à raison de 0,1 à 5%, de préférence de 0,5 à 2% en poids de la composition.

**19.** Composition selon l'une quelconque des revendications 6 à 18, dans laquelle la silice finement divi- sée présente une granulométrie de 0,001 à 0,050 µm, de préférence de 0,010 à 0,020 µm.

**20.** Composition selon l'une quelconque des revendications 6 à 19, dans laquelle la silice finement divisée est la silice fumée.

**21.** Composition selon l'une quelconque des revendications 6 à 20, dans laquelle la composition de cire de suspension est présente à raison de 1,5 à 7% en poids de la composition.

**22.** Composition selon l'une quelconque des revendications 6 à 21, dans laquelle l'ester de la composition de cire de suspension est l'adipate de dioctyle.

**23.** Composition selon l'une quelconque des revendications 6 à 22, dans laquelle la cire de la suspension de cire en suspension est une cire synthétique, une cire naturelle, une composition analogue à une cire ou une combinaison de celles-ci et elle est de préférence choisie parmi la cire d'huile de ricin, la cire d'abeilles, la cire de carnauba, la cire d 'ozokérite, la lanoline hydrogénée, le beurre de cacao hydrogéné, le polyéthylène et leurs mélanges.

**24.** Composition selon l'une quelconque des revendications 6 à 23, dans laquelle la silicone volatile de la composition de cire de suspension présente une viscosité de 0,005 $cm^2/s$ (0,5 cs) à 0,06 $cm^2/s$ (6 cs) et un point d'ébullition à 25°C sous pression atmosphérique de 100 à 250°C.

**25.** Composition selon l'une quelconque des revendications 6 à 24, dans laquelle l'hydrocarbure volatil de la composition de cire de suspension est le même que le véhicule hydrocarboné volatil défini dans les revendications 16 et 17.

**26.** Composition selon l'une quelconque des revendications 6 à 25, dans laquelle la composition de cire en suspension comprend de 25 à 35% en poids de cire d'huile de ricin ; de 30 à 45% en poids d'un hydrocarbure volatil de formule structurale :

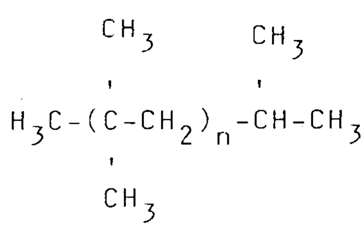

dans laquelle n est un nombre compris entre 2 et 5 ;
et de 25 à 40% en poids d'adipate de dioctyle.

**27.** Composition selon l'une quelconque des revendications 6 à 26, comprenant en outre environ 0,1 à 30% en poids d'un cosolvant.

**28.** Composition selon la revendication 27, dans laquelle le cosolvant est une silicone non volatile ; un benzoate d'un alcool contenant 8 à 30 atomes de carbone ; un polyol de haute masse moléculaire ; un tensioactif soluble dans l'huile ou une combinaison de ceux-ci.

29. Composition selon la revendication 28, dans laquelle la silicone non volatile est une diméthicone, un fluide diphénylsiloxane ou une combinaison de ceux-ci ; le polyol de haute masse moléculaire est de préférence choisi parmi le polysorbiate 60, un polypropylène-glycol et une combinaison de ceux-ci et le tensioactif soluble dans l'huile est de préférence une décylpyrrolidone, un nonoxynol-2 ou un mélange de ceux-ci.

30. Composition selon l'une quelconque des revendications 6 à 20, comprenant en outre de 0,1 à 20% en poids d'une argile à trois couches quaternisée, exfoliée avec un solvant polaire, de préférence une montmorillonite quaternisée exfoliée avec le propylène-glycol.

31. Composition selon l'une quelconque des revendications 6 à 30, comprenant en outre des ingrédients facultatifs choisis parmi 0,5 à 2% en poids d'un agent siccatif ; 3 à 10% en poids d'un émollient ; de 0,5 à 10% en poids d'un agent de fragrance ; et des combinaisons de ceux-ci ; dans laquelle l'agent siccatif est de préférence l'octényl-succinate amylacé d'aluminium et l'émollient est de préférence choisi parmi un ester gras, un alcool gras ; une huile minérale ; un copolymère de polyéther-siloxane et des combinaisons de ceux-ci.

32. Procédé de distribution d'un composé cosmétique localement actif, qui consiste :
à mettre en contact avec la peau ou les cheveux une quantité suffisante d'une composition telle que définie dans les revendications 1 à 31.

33. Procédé de fabrication d'une composition stable localement efficace sous forme d'un liquide, d'une crème ou d'une pâte pour distribution locale d'un composé localement actif, qui consiste :
à préparer une composition de cire de suspension comprenant de 25 à 35% en poids d'une cire ; de 30 à 45% en poids d'un solvant volatil choisi parmi une silicone volatile, un hydrocarbure volatil ou un mélange de ceux-ci, solvant dont le point d'ébullition est de 150 à 250°C s'il est un polydiméthylsiloxane et de 100 à 300°C s'il est un hydrocarbure ; et de 25 à 40% en poids d'un ester contenant au moins 10 atomes de carbone ; à préparer une dispersion de silicone comprenant de 0,1 à 15 parties en poids d'une silice finement divisée et de 20 à 95 parties en poids d'un véhicule liquide volatil ;
à combiner 1 à 10 parties en poids de la composition de cire de suspension avec 0,01 à 30 parties en poids d'un composé localement actif avec une dispersion de silice pour former un mélange localement actif; et
à incorporer intimement le mélange localement actif pour former une composition homogène localement efficace.

**Revendications pour les Etats contractants suivants : ES, GR**

1. Procédé de production d'une composition localement efficace sous forme d'un liquide, d'une crème ou d'une pâte pour application à la peau ou aux cheveux, qui consister à mélanger :
   (a) de 0,01 à 30% en poids d'un composé localement actif ;
   (b) de 20 à 99% en poids d'un véhicule liquide volatil dont le point d'ébullition est compris entre 150 et 250°C s'il est un polydiméthylsiloxane et entre 100 et 300°C s'il est un hydrocarbure ;
   (c) de 0,1 à 15% en poids d'une silice finement divisée ;
   (d) de 0,25 à 3,5% en poids d'une cire ;
   (e) de 0,25 à 4,0% en poids d'un ester contenant au moins 10 atomes de carbone.

2. Procédé selon la revendication 1, dans lequel le composé localement actif est un composé anti-perspirant, un composé antipelliculaire, un composé anti-fongique, un composé anti-inflammatoire, un composé filtre solaire, un composé anti-bactérien, un composé anesthésique local, un médicament local, un composé dermatologique ou un composé analgésique.

3. Procédé selon la revendication 1, dans lequel le véhicule liquide volatil est une silicone volatile cyclique, une silicone volatile linéaire, un hydrocarbure volatil ou une combinaison de ceux-ci.

4. Procédé selon la revendication 1, dans lequel la cire est une cire synthétique, une cire naturelle ou une combinaison de celles-ci.

5. Procédé selon la revendication 1, dans lequel l'ester contient de 12 à 32 atomes de carbone.

6. Procédé selon la revendication 1, qui consiste à mélanger les composants (a) à (c) de la revendication 1 avec :
(d) de 1 à 10% en poids d'une composition de cire de suspension dans laquelle la composition de cire de suspension comprend de 25 à 35% en poids d'une cire ; de 30 à 45% en poids d'une silicone volatile ou d'un hydrocarbure volatil ; et de 25 à 40% en poids d'un ester contenant au moins 10 atomes de carbone.

7. Procédé selon la revendication 6, dans lequel le composé localement actif est présent à raison de 1 à 25%, de préférence de 5 à 20%.

8. Procédé selon la revendication 7, dans lequel le composé antiperspirant est un sel astringent choisi parmi les sels d'aluminium, les sels de zirconium, les sels mélangés d'aluminium et de zirconium et leurs mélanges.

9. Procédé selon la revendication 8, dans lequel le composé antiperspirant est choisi parmi le tetrachlorhydrex-glycinate de zirconium et aluminium (complexe de coordination de tetrachlorhydrate d'aluminium et zirconium et de glycine), le chlorhydrate d'aluminium $(Al_2(OH)_5)_n$ x nCl, le chlorhydrate de zirconium (polymère minéral amorphe soluble formé par neutralisation partielle de chlorure de zirconyle) et le chlorhydrex d'aluminium.

10. Procédé selon l'une quelconque des revendications 6 à 9 , dans lequel le véhicule liquide volatil est présent à raison de 30 à 75% en poids.

11. Procédé selon l'une quelconque des revendications 1 à 10, dans lequel la silicone volatile cyclique présente une viscosité à 25°C comprise entre 0,02 $cm^2/s$ (2 cs) et 0,06 $cm^2/s$ (6 cs) et un point d'ébullition à 25°C et 760 mmHg (1 atm) compris entre 150 et 250°C.

12. Procédé selon la revendication 11, dans lequel la silicone volatile cyclique est une cyclométhicone (composé cyclique de diméthylpolysiloxane) de formule :

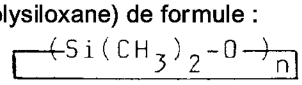 ;

13. Procédé selon la revendication 12, dans lequel la cyclométhicone est choisie parmi l'hexaméthylcyclotrisiloxane ; l'octaméthylcyclotetrasiloxane ; le decaméthylcyclopentasiloxane ; le dodecaméthylcyclohexasiloxane et leurs mélanges.

14. Procédé selon la revendication 10, dans lequel la silicone volatile linéaire présente une viscosité à 25°C comprise entre 0,005 $cm^2/s$ (0,5 cs) et 0,05 $cm^2/s$ (5 cs) et un point d'ébullition à 25°C sous 760 mmHg (1 atm) de 100 à 250°C.

15. Procédé selon la revendication 14, dans lequel la silicone volatile est choisie parmi l'hexaméthyldisiloxane, l'octaméthyltrisiloxane, le decaméthyltetrasiloxane, le dodecaméthylpentasiloxane et leurs combinaisons.

16. Procédé selon la revendication 10, dans lequel l'hydrocarbure volatil comprend de 12 à 24 atomes de carbone et son point d'ébullition à 25°C et 760 mmHg (1 atm) est de 100 à 300°C.

17. Procédé selon la revendication 16, dans lequel l'hydrocarbure volatil répond à la formule structurale :

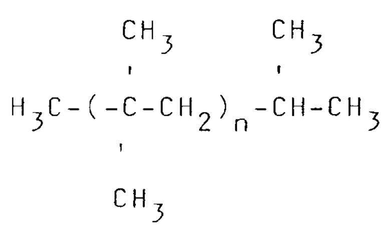

dans laquelle n est compris entre 2 et 5, et leurs mélanges.

**18.** Procédé selon l'une quelconque des revendications 6 à 17, dans lequel la silice finement divisée est présente à raison de 0,1 à 5%, de préférence de 0,5 à 2% en poids de la composition.

**19.** Procédé selon l'une quelconque des revendications 6 à 18, dans lequel la silice finement divisée présente une granulométrie de 0,001 à 0,050 µm, de préférence de 0,010 à 0,020 µm.

**20.** Procédé selon l'une quelconque des revendications 6 à 19, dans lequel la silice finement divisée est la silice fumée.

**21.** Procédé selon l'une quelconque des revendications 6 à 20, dans lequel la composition de cire de suspension est présente à raison de 1,5 à 7% en poids de la composition.

**22.** Procédé selon l'une quelconque des revendications 6 à 21, dans lequel l'ester de la composition de cire de suspension est l'adipate de dioctyle.

**23.** Procédé selon l'une quelconque des revendications 6 à 22, dans lequel la cire de la suspension de cire en suspension est une cire synthétique, une cire naturelle, une composition analogue à une cire ou une combinaison de celles-ci et elle est de préférence choisie parmi la cire d'huile de ricin, la cire d'abeilles, la cire de carnauba, la cire d'ozokérite, la lanoline hydrogénée, le beurre de cacao hydrogéné, le polyéthylène et leurs mélanges.

**24.** Procédé selon l'une quelconque des revendications 6 à 23, dans lequel la silicone volatile de la composition de cire de suspension présente une viscosité de 0,005 $cm^2/s$(0,5 cs) à 0,06 $cm^2/s$ (6 cs) et un point d'ébullition à 25°C sous pression atmosphérique de 100 à 250°C.

**25.** Procédé selon l'une quelconque des revendications 6 à 24, dans lequel l'hydrocarbure volatil de la composition de cire de suspension est le même que le véhicule hydrocarboné volatil défini dans les revendications 16 et 17.

**26.** Procédé selon l'une quelconque des revendications 6 à 25, dans lequel la composition de cire en suspension comprend de 25 à 35% en poids de cire d'huile de ricin ; de 30 à 45% en poids d'un hydrocarbure volatil de formule structurale :

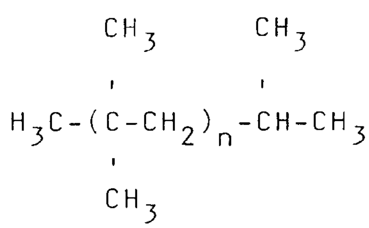

dans laquelle n est un nombre compris entre 2 et 5 ; et de 25 à 40% en poids d'adipate de dioctyle.

**27.** Procédé selon l'une quelconque des revendications 6 à 26, comprenant en outre environ 0,1 à 30% en poids d'un cosolvant.

**28.** Procédé selon la revendication 27, dans lequel le cosolvant est une silicone non volatile ; un benzoate d'un alcool contenant 8 à 30 atomes de carbone ; un polyol de haute masse moléculaire ; un tensioactif soluble dans l'huile ou une combinaison de ceux-ci.

**29.** Procédé selon la revendication 28, dans lequel la silicone non volatile est une diméthicone, un fluide diphénylsiloxane ou une combinaison de ceux-ci ; le polyol de haute masse moléculaire est de préférence choisi parmi le polysorbiate 60, un polypropylène-glycol et une combinaison de ceux-ci ; et le tensioactif soluble dans l'huile est de préférence une décylpyrrolidone, un nonoxynol-2 ou un mélange de ceux-ci.

**30.** Procédé selon l'une quelconque des revendications 6 à 28, comprenant en outre de 0,1 à 20% en poids d'une argile à trois couches quaternisée, exfoliée avec un solvant polaire, de préférence une montmorillonite quaternisée exfoliée avec le propylène-glycol.

**31.** Procédé selon l'une quelconque des revendications 6 à 30, comprenant en outre des ingrédients facultatifs choisis parmi 0,5 à 2% en poids d'un agent siccatif 3 à 10% en poids d'un émollient ; de 0,5 à 10% en poids d'un agent de fragrance ; et des combinaisons de ceux-ci ; dans lequel l'agent siccatif est de préférence l'octényl-succinate amylacé d'aluminium et l'émollient est de préférence choisi parmi un ester gras, un alcool gras ; une huile minérale ; un copolymère de polyéther-siloxane et des combinaisons de ceux-ci.

**32.** Procédé de distribution d'un composé cosmétique localement actif, qui consiste :

à mettre en contact avec la peau ou les cheveux une quantité suffisante d'une composition obtenue par un procédé selon l'une quelconque des revendications 1 à 31.

**33.** Procédé de fabrication d'une composition stable localement efficace sous forme d'un liquide, d'une crème ou d'une pâte pour distribution locale d'un composé localement actif, qui consiste :

à préparer une composition de cire de suspension comprenant de 25 à 35% en poids d'une cire ; de 30 à 45% en poids d'un solvant volatil choisi parmi une silicone volatile, un hydrocarbure volatil ou un mélange de ceux-ci, solvant dont le point d'ébullition est de 150 à 250°C s'il est un polydiméthylsiloxane et de 100 à 300°C s'il est un hydrocarbure ; et de 25 à 40% en poids d'un ester contenant au moins 10 atomes de carbone ; à préparer une dispersion de silicone comprenant de 0,1 à 15 parties en poids d'une silice finement divisée et de 20 à 95 parties en poids d'un véhicule liquide volatil ;

à combiner 1 à 10 parties en poids de la composition de cire de suspension avec 0,01 à 30 parties en poids d'un composé localement actif avec une dispersion de silice pour former un mélange localement actif; et

à incorporer intimement le mélange localement actif pour former une composition homogène localement efficace.